# EUROPEAN PATENT APPLICATION

(11) **EP 4 116 420 A1**
(43) Date of publication of application: **11.01.2023**
(21) Application number: 21767174.2
(22) Date of filing: 08.03.2021
(51) Int. Cl.: C12N 15/113, C07K 16/30, A61K 39/395

(54) **USE OF GP73 INHIBITOR IN PREPARATION OF MEDICINE FOR TREATING DIABETES**

(30) Priority: 08.03.2020 CN 202010154792
(71) Applicant: Beijing Sungen Biomedical Technology Co. Ltd., Beijing 100000 (CN)
(72) Inventor: LIN, Changqing, Daxing District, Beijing 100000 (CN); SUN, Zhiwei, Daxing District, Beijing 100000 (CN); GAO, Qi, Daxing District, Beijing 100000 (CN); QIE, Shuang, Beijing 100000 (CN); XU, Lei, Beijing 100000 (CN); LI, Jing, Beijing 100000 (CN); LIN, Jianbo, Beijing 100000 (CN); ZHU, Hengqi, Beijing 100000 (CN); ZHENG, Fei, Beijing 100000 (CN); LIU, Xuechao, Beijing 100000 (CN)
(74) Representative: LLR
(86) International application number: PCT/CN2021/079613
(87) International publication number: WO 2021/180047

(57) **Abstract**

Embodiments of the present invention relate to use of a GP73 inhibitor in preparation of a drug for treating diabetes. In the embodiments of the present invention, the inventor finds that GP73 plays a key role in blood glucose regulation, and in particular, finds that soluble GP73 can specifically bind to glucagon to form a complex, enhances the blood glucose-rising function and gluconeogenesis function of glucagon and prolongs the half-life of glucagon; and finds soluble GP73 can activate the glucose production in liver and/or kidney and a gluconeogenesis signaling pathway in a glucagon-independent manner. Based on the blood glucose regulation effect of the GP73 described above, the inventor also proves through animal experiments: the GP73 inhibitor can reduce the blood glucose level and glycated hemoglobin level of diabetic mice and have a protective effect on islet β cells, and thereby having the effect of treating diabetes.

## Description

### Cross-reference

The present invention claims priority to Chinese Patent Application with the application number of 202010154792.3 filed with Patent Office of the People's Republic of China and titled "USE OF GP73 INHIBITOR IN PREPARATION OF DRUG FOR TREATING DIABETES", which is incorporated herein by reference in its entirety.

### Technical Field

The present invention relates to the field of biomedicine, in particular to use of a GP73 inhibitor in preparation of a drug for treating diabetes.

### Background Art

It is estimated by the International Diabetes Federation that in 2017, about 425 million (8.8%) of the world's population aged 20-79 years were living with diabetes, and 4 million people died due to diabetes, accounting for 10.7% of the global death toll. China has the highest number of diabetics in the world, i.e., about 114.4 million diabetics. This figure is on the rise year by year and is expected to increase to at least 629 million diabetics aged 20-79 in the world by 2045.

Diabetes, a metabolic disorder characterized by hyperglycemia, is mainly classified into four types: type I diabetes, type II diabetes, gestational diabetes and other special types of diabetes. Type I diabetes, insulin-dependent diabetes, mainly results from destruction of islet β cells mediated by an autoimmune response, accounting for 5%-10% of the total number of diabetics. Type I diabetes develops in six stages: (1) genetic susceptibility; (2) autoimmune responses initiated by certain environmental factors; (3) normal insulin secretion function in the active stage of autoimmune responses; (4) autoimmune responses existing persistently, and progressively decreased insulin secretion function; (5) part of the insulin secretion function retained after the onset of clinical diabetes; and (6) completely destructed pancreatic β. Type II diabetes is mainly caused by insulin deficiency and insulin resistance in the body, accounting for 90%-95% of the total number of diabetics. Type II diabetes develops in 4 stages: (1) genetic susceptibility; (2) hyperinsulinemia and/or insulin resistance; (3) impaired glucose tolerance; and (4) clinical diabetes.

The current research results show that excess glucagon (GCG) is a key factor of the onset of diabetes. The main evidence is that: (1) in the case of insulin deficiency, glucagon increases the production of hepatic glucose and ketone bodies; (2) diabetics with poor control over various types of blood glucose have hyperglucagonemia; (3) destructing all β cells of mice with glucagon receptor defect does not lead to diabetes; and (4) infusion of anti-insulin serum to the pancreas of mice with glucagon receptor defect obviously leads to hyperglucagonemia, and insulin in the pancreas islet has a sustained paracrine inhibitory effect on secretion of glucagon. Glucagon, a peptide hormone mainly secreted by pancreatic islet cells, is a straight-chain polypeptide consisting of 29 amino acids with a molecular weight of 3485 Da. Glucagon interacts with its receptor (GCGR) to promote glycogenolysis and gluconeogenesis through signaling pathways such as cAMP, AMPK and JNK, thereby increasing the concentration of glucose in the blood. The liver, brain, gastrointestinal tract, kidney, adipose tissue, heart and other organs are target organs of glucagon. Among them, the liver is the main target organ for raising the blood glucose.

Therefore, diabetes is a bihormonal chaotic pancreatic disease with insulin deficiency, insulin resistance and excess glucagon. At present, there are many kinds of drugs for treating diabetes, but these drugs still focus on insulin and thus hardly change the gradual deterioration of blood glucose control, and the blood glucose of more than one third of diabetics has not been well controlled. The onset of diabetes is the result of a combination of multiple genetic susceptibility and multiple environmental factors, which leads to the heterology and progressive pathological changes of the disease, and thus an existing treatment method has obvious limitations in treating diabetes. In addition, diabetes-related complications and comorbidities also limit the use of some drugs. Meantime, when the treatment time is long, some drugs will lose their treatment effects, so it is of great practical significance to continuously develop new diabetes treatment drugs.

Golgi protein 73 (GP73), a type II transmembrane protein located in a Golgi complex, is also known as Golgi membrane protein 1 (GOLM1) or Golgi phosphorprotein 2 (GOLPH2). It is located on chromosome 9 and has a length of 3042 bp. Its gene has two methionine codons in the same reading frame, and the two-methionine codons are separated by 10 codons and transcribe 400 or 391 amino acid products respectively. The structure of GP73 is mainly divided into five parts: cytoplasmic domains 1-12 of the N-terminal, transmembrane domains 12-35, coiled-coil domains 36-205, amorphous domains 206-348 and acidic fragment regions 349-401. Except that the amorphous domain is a variable region, other domains are highly conservative. A proprotein convertase (PC) cleavage site is presented near the amino acid 55 of GP73. After being cleaved by PC, full-length GP73 is released from the Golgi complex and secreted into the blood circulatory system. The GP73 fragment released into the blood is called soluble GP73. GP73 is hardly expressed in normal liver tissue, but is expressed in almost all liver cells, especially in the periphery of connective tissue and cirrhotic nodules, when the body has a liver disease due to various causes. In more than 70% of patients with liver cancer, GP73 protein has significantly up-regulated expression level in serum and liver tissue, which is 3-5 times of that in normal tissue. Therefore, serum GP73 is considered as a serological tumor marker for effective diagnosis of liver cancer. In addition, GP73 has significantly up-regulated expression in esophageal cancer, breast cancer, the prostate tissue and urine of patients with prostate cancer, bladder cancer, cervical cancer and other tumors.

Although the abnormally high expression of GP73 is closely related to various tumors, the biological function of GP73 is still unclear. Little is known about the extracellular function of soluble GP73. At present, only one report shows that GP73 in serum mediates the transfer of endoplasmic reticulum stress between hepatocytes and immune cells, and this cascade amplification effect induces the recruitment of tumor-associated macrophages and causes immune tolerance in the tumor microenvironment.

The information disclosed in the Background is only for enhancing understanding of the general background of the present invention and should not be taken as admitting or implying in any form that this information forms the prior art known to those skilled in the art.

### Summary of the Invention

### Object of Invention

In the embodiments of the present invention, the inventor finds that GP73 plays a key role in blood glucose regulation, and in particular, finds that soluble GP73 can specifically bind to glucagon to form a complex, enhances the blood-glucose-rising function and gluconeogenesis function of glucagon and prolongs the half-life of glucagon; and soluble GP73 can activate the glucose production in liver and/or kidney and a gluconeogenesis signaling pathway in a glucagon-independent manner. The inventor also finds that soluble GP73 can rise fasting blood glucose in mice, and induce abnormal glucose tolerance and pyruvate tolerance. Based on the blood glucose regulation effect of GP73 described above, the inventor also proves that through animal experiments: the GP73 inhibitor can reduce the blood glucose level and glycated hemoglobin level of diabetic mice and have a protective effect on islet β cells, and thereby having the effect of treating diabetes.

The present invention provides the following technical schemes.

The first aspect of the present invention provides use of a GP73 inhibitor in preparation of a drug for treating diabetes and complications thereof.

The second aspect of the present invention provides a drug for treating diabetes and complications thereof, wherein the drug comprises a GP73 inhibitor as an active ingredient.

The third aspect of the present invention provides a method for treating diabetes and complications thereof, wherein the method comprises the following steps: administering an effective dose of GP73 inhibitor to a subject with diabetes.

According to the above use, drug and method in a possible implementation manner, the diabetes comprises type I diabetes, type II diabetes and gestational diabetes.

According to the above use, drug and method in a possible implementation manner, treating diabetes comprises any one or more of the following: (1) reducing fasting blood glucose and/or postprandial blood glucose; (2) improving glucose tolerance; (3) protecting islet α cells and/or islet β cells; (4) reducing the glucose-rising ability and/or gluconeogenesis ability of glucagon; (5) shortening the half-life of glucagon; and (6) reducing the non-insulin-dependent glucose-rising effect and gluconeogenesis function of GP73 itself.

According to the above use, drug and method in a possible implementation manner, the complications thereof comprise any one or more of the following: diabetic nephropathy, diabetic eye complications, diabetic foot and diabetic peripheral neuropathy, wherein the diabetic eye complications comprise one or more of the following: diabetic retinopathy, uveitis related to diabetes and diabetic cataract.

According to the above use, drug and method in a possible implementation manner, the GP73 inhibitor comprises: polypeptides, protein, nucleic acid sequences or small molecule compounds that down-regulate the level, activity, function and/or stability of GP73. Optionally, the polypeptides, protein, nucleic acid sequences or small molecule compounds that down-regulates the level, activity, function and/or stability of GP73 have one or more of the following properties: (1) inhibiting transcription, correct cutting and/or translation of genes encoding the GP73; (2) inhibiting or hindering the binding of the GP73 to receptors and/or ligands in the body; (3) inhibiting or hindering the interaction between the GP73 and specific interacting molecules in the body; and (4) shortening the half-life of the GP73 in the body. Further optionally, the GP73 inhibitor comprises: one or more of an anti-GP73 monoclonal antibody or an antibody fragment comprising an antigen-binding site thereof, a fusion protein of the anti-GP73 monoclonal antibody or the antibody fragment comprising the antigen-binding site thereof, and a nucleic acid sequence that specifically inhibits the GP73.

According to the above use, drug and method in a possible implementation manner, the GP73 is selected from one or more of the following: natural or recombinant full-length GP73, a GP73 fragment, a GP73 mutant or modified GP73 existing in the body or isolated in vitro; optionally, the GP73 is selected from full-length GP73 or GP73 excluding amino acids 1-55.

According to the above use, drug and method in a possible implementation manner, the anti-GP73 monoclonal antibody is selected from: one or more of a monoclonal antibody produced by hybridoma cells, a monoclonal antibody screened by an antibody library, a monoclonal antibody produced by single cell PCR, a genetically engineered monoclonal antibody, a heterologous antibody, a chimeric antibody, a humanized antibody, an fully-human antibody, a nanobody and a heavy chain antibody.

According to the above use, drug and method in a possible implementation manner, the antibody fragment is selected from: one or more of Fab, Fab-SH, Fv, scFv, F(ab')₂, DsFv, Diabody, Minibody, Tribody, Sc(Fv)₂, [Sc(Fv)₂]₂ and (ScFv-SA)₄.

According to the above use, drug and method in a possible implementation manner, the nucleic acid that specifically inhibits GP73 comprises one or more of siRNA, shRNA, microRNA, antisense oligonucleotide, miRNA and a nucleic acid aptamer. Optionally, the siRNA that specifically inhibits GP73 is selected from one or more of nucleotide sequences shown in SEQ ID NO: 1 to SEQ ID NO: 9, or is selected from a sequence having at least 60%, 70%, 80% and 90% homology to any one of the nucleotide sequence shown in SEQ ID NO: 1 to SEQ ID NO: 9. Further optionally, the siRNA that specifically inhibits GP73 is selected from a nucleotide sequence shown in SEQ ID NO: 4 or a sequence having at least 60%, 70%, 80% and 90% homology thereto.

According to the above use, drug and method in a possible implementation manner, the drug for treating diabetes and complications thereof also comprises other drugs for treating diabetes. The method for treating diabetes and complications thereof is used in combination with the GP73 inhibitor and other drugs for treating diabetes.

According to the above use, drug and method in a possible implementation manner, the other drugs for treating diabetes are selected from one or more of insulin, dimethyl biguanide, sulfonylurea hypoglycemic drugs, α- glycosidase inhibitors, thiazolidinediones, dipeptidyl peptidase 4 (DPP4) inhibitors, glucagon-like peptide-1 (GLP-1) analogs and SGLT2 inhibitors.

According to the above use and drug in a possible implementation manner, the drug also comprises at least one pharmaceutically acceptable excipient.

According to the above use, drug and method in a possible implementation manner, the drug is applied through one or more of intravenous injection, intramuscular injection, subcutaneous injection and oral administration.

According to the above method in a possible implementation manner, the subject is selected from one or more of human, mice, rats, monkeys, rabbits, pigs and dogs.

The fourth aspect of the present invention provides use of a GP73 inhibitor in preparation of a drug for inhibiting glucagon.

The fifth aspect of the present invention provides a drug for inhibiting glucagon, wherein the drug comprises a GP73 inhibitor.

The sixth aspect of the present invention provides a method for inhibiting glucagon, wherein the method comprises the following steps: administering an effective dose of GP73 inhibitor to a subject in need of glucagon inhibition.

According to the above use, drug and method in a possible implementation manner, inhibiting glucagon comprises any one or more of the following: (1) shortening the half-life of glucagon; and (2) reducing the glucose-rising ability and/or gluconeogenesis ability of glucagon.

According to the above use, drug and method in a possible implementation manner, the GP73 inhibitor comprises: polypeptides, protein, nucleic acid sequences or small molecule compounds that down-regulate the level, activity, function and/or stability of GP73. Optionally, the polypeptides, protein, nucleic acid sequences or small molecule compounds that down-regulates the level, activity, function and/or stability of GP73 have one or more of the following properties: (1) inhibiting transcription, correct cutting and/or translation of genes encoding the GP73; (2) inhibiting or hindering the binding of the GP73 to receptors and/or ligands in the body; (3) inhibiting or hindering the interaction between the GP73 and specific interacting molecules in the body; and (4) shortening the half-life of the GP73 in the body. Further optionally, the GP73 inhibitor comprises: one or more of an anti-GP73 monoclonal antibody or antibody fragment comprising an antigen-binding site thereof, a fusion protein of the anti-GP73 monoclonal antibody or antibody fragment comprising the antigen-binding site thereof, and a nucleic acid sequence that specifically inhibits the GP73.

According to the above use, drug and method in a possible implementation manner, the GP73 is selected from one or more of the following: natural or recombinant full-length GP73, a GP73 fragment, a GP73 mutant or modified GP73 existing in the body or isolated in vitro; optionally, the GP73 is selected from full-length GP73 or GP73 excluding amino acids 1-55.

According to the above use, drug and method in a possible implementation manner, the anti-GP73 monoclonal antibody is selected from: one or more of a monoclonal antibody produced by hybridoma cells, a monoclonal antibody screened by an antibody library, a monoclonal antibody produced by single cell PCR, a genetically engineered monoclonal antibody, a heterologous antibody, a chimeric antibody, a humanized antibody, an fully-human antibody, a nanobody and a heavy chain antibody.

According to the above use, drug and method in a possible implementation manner, the antibody fragment is selected from: one or more of Fab, Fab-SH, Fv, scFv, F(ab')₂, DsFv, Diabody, Minibody, Tribody, Sc(Fv)₂, [Sc(Fv)₂]₂ and (ScFv-SA)₄.

According to the above use, drug and method in a possible implementation manner, the nucleic acid that specifically inhibits GP73 comprises one or more of siRNA, shRNA, microRNA, antisense oligonucleotide, miRNA and a nucleic acid aptamer. Optionally, the siRNA that specifically inhibits GP73 is selected from one or more of nucleotide sequences shown in SEQ ID NO: 1 to SEQ ID NO: 9, or is selected from a sequence having at least 60%, 70%, 80% and 90% homology to any one of the nucleotide sequence shown in SEQ ID NO: 1 to SEQ ID NO: 9. Further optionally, the siRNA that specifically inhibits GP73 is selected from a nucleotide sequence shown in SEQ ID NO: 4 or a sequence having at least 60%, 70%, 80% and 90% homology thereto.

According to the above use and drug in a possible implementation manner, the drug also comprises at least one pharmaceutically acceptable excipient.

According to the above use, drug and method in a possible implementation manner, the drug is applied through one or more of intravenous injection, intramuscular injection, subcutaneous injection and oral administration.

According to the above method in a possible implementation manner, the subject is selected from one or more of human, mice, rats, monkeys, rabbits, pigs and dogs.

The seventh aspect of the present invention provides a GP73-glucagon complex, wherein GP73 binds to glucagon.

According to the above GP73-glucagon complex in a possible implementation manner, the GP73 is selected from one or more of the following: natural or recombinant full-length GP73, a GP73 fragment, a GP73 mutant or modified GP73 existing in the body or isolated in vitro; optionally, the GP73 is selected from full-length GP73 or GP73 excluding amino acids 1-55.

According to the above GP73-glucagon complex in a possible implementation manner, the GP73 is derived from one or more of human, mice, rats, monkeys, rabbits, pigs and dogs.

The eighth aspect of the present invention provides a method for determining a binding epitope of GP73 to glucagon, comprising the following steps: the binding epitope of GP73 to glucagon is determined by one or more of complex crystallization analysis method, epitope determination site excision method, hydrogen tritium exchange method and peptide-panning method.

The ninth aspect of the present invention provides a method for determining the inhibitory effect of a GP73 inhibitor on inhibiting formation of a GP73-glucagon complex, comprising any one of the following two methods:
Method 1:
   after incubating a candidate GP73 inhibitor with GP73, the resulting mixture and/or complex binds to glucagon, and
   comparing the binding abilities of the GP73 to the glucagon before and after incubating with the candidate GP73 inhibitor;
Method 2:
   comparing the binding abilities of GP73 to glucagon before and after incubating with the candidate GP73 inhibitor by using computer simulation.

According to the method for determining the inhibitory effect of a GP73 inhibitor on inhibiting formation of a GP73-glucagon complex in a possible implementation manner, the candidate GP73 inhibitor is derived from one or more selected from the group consisting of: a hybridoma cell, a B cell, a memory B cell, an antibody library, a compound library, a GP73 analog and a glucagon analog.

According to the method for determining the inhibitory effect of a GP73 inhibitor on inhibiting formation of a GP73-glucagon complex in a possible implementation manner, the method for determining the binding ability of GP73 to glucagon comprises one or more of the following methods: a surface plasmon resonance (SPR) assay, microscale thermophoresis (MST) assay and a competitive ELISA assay.

The tenth aspect of the present invention provides use of a GP73 detection reagent in preparation of a reagent for detecting diabetes.

The eleventh aspect of the present invention provides a reagent for detecting diabetes, the reagent comprising a GP73 detection reagent.

According to the above use and reagent in a possible implementation manner, the diabetes comprises type I diabetes, type II diabetes and gestational diabetes.

According to the above use and reagent in a possible implementation manner, the GP73 detection reagent comprises a reagent for detecting soluble GP73 in serum.

The twelfth aspect of the present invention provides use of a GP73 inhibitor in preparation of a drug for a gluconeogenesis signaling pathway inhibitor.

The thirteenth aspect of the present invention provides a drug for inhibiting a gluconeogenesis signaling pathway, wherein the drug comprises a GP73 inhibitor.

The fourteenth aspect of the present invention provides a method for inhibiting a gluconeogenesis signaling pathway, comprising the following steps: administering an effective dose of GP73 inhibitor to a subject in need of inhibiting the gluconeogenesis signaling pathway.

According to the above use, drug and method in a possible implementation manner, inhibiting a gluconeogenesis signaling pathway comprises any one or more of the following: (1) inhibiting gluconeogenesis of hepatocytes to produce glucose; (2) down-regulating the expression levels of key gluconeogenesis enzymes Pcx, Pckl and G6pc; and (3) down-regulating PKA phosphorylation level and kinase activity.

According to the above use, drug and method in a possible implementation manner, the GP73 inhibitor comprises: polypeptides, protein, nucleic acid sequences or small molecule compounds that down-regulate the level, activity, function and/or stability of GP73. Optionally, the polypeptides, protein, nucleic acid sequences or small molecule compounds that down-regulates the level, activity, function and/or stability of GP73 have one or more of the following properties: (1) inhibiting transcription, correct cutting and/or translation of genes encoding the GP73; (2) inhibiting or hindering the binding of the GP73 to receptors and/or ligands in the body; (3) inhibiting or hindering the interaction between the GP73 and specific interacting molecules in the body; and (4) shortening the half-life of the GP73 in the body. Further optionally, the GP73 inhibitor comprises: one or more of an anti-GP73 monoclonal antibody or an antibody fragment comprising an antigen-binding site thereof, a fusion protein of the anti-GP73 monoclonal antibody or the antibody fragment comprising the antigen-binding site thereof, and a nucleic acid sequence that specifically inhibits the GP73.

According to the above use, drug and method in a possible implementation manner, the GP73 is selected from one or more of the following: natural or recombinant full-length GP73, a GP73 fragment, a GP73 mutant or modified GP73 existing in the body or isolated in vitro; optionally, the GP73 is selected from full-length GP73 or GP73 excluding amino acids 1-55.

According to the above use, drug and method in a possible implementation manner, the anti-GP73 monoclonal antibody is selected from: one or more of a monoclonal antibody produced by hybridoma cells, a monoclonal antibody screened by an antibody library, a monoclonal antibody produced by single cell PCR, a genetically engineered monoclonal antibody, a heterologous antibody, a chimeric antibody, a humanized antibody, an fully-human antibody, a nanobody and a heavy chain antibody.

According to the above use, drug and method in a possible implementation manner, the antibody fragment is selected from: one or more of Fab, Fab-SH, Fv, scFv, F(ab')₂, DsFv, Diabody, Minibody, Tribody, Sc(Fv)₂, [Sc(Fv)₂]₂ and (ScFv-SA)₄.

According to the above use, drug and method in a possible implementation manner, the nucleic acid that specifically inhibits GP73 comprises one or more of siRNA, shRNA, microRNA, antisense oligonucleotide, miRNA and a nucleic acid aptamer. Optionally, the siRNA that specifically inhibits GP73 is selected from one or more of nucleotide sequences shown in SEQ ID NO: 1 to SEQ ID NO: 9, or is selected from a sequence having at least 60%, 70%, 80% and 90% homology to any one of the nucleotide sequence shown in SEQ ID NO: 1 to SEQ ID NO: 9. Further optionally, the siRNA that specifically inhibits GP73 is selected from a nucleotide sequence shown in SEQ ID NO: 4 or a sequence having at least 60%, 70%, 80% and 90% homology thereto.

According to the above use and drug in a possible implementation manner, the drug also comprises at least one pharmaceutically acceptable excipient.

According to the above use, drug and method in a possible implementation manner, the drug is applied through one or more of intravenous injection, intramuscular injection, subcutaneous injection and oral administration.

According to the above method in a possible implementation manner, the subject is selected from one or more of human, mice, rats, monkeys, rabbits, pigs and dogs.

### Beneficial Effects

(1) In the embodiments of the present invention, the inventor finds that GP73 plays a key role in blood glucose regulation, and in particular, finds that soluble GP73 can specifically bind to glucagon to form a complex, enhances the blood-glucose-rising function and gluconeogenesis function of glucagon and prolongs the half-life of glucagon; and soluble GP73 can activate the glucose production in liver and a gluconeogenesis signaling pathway in a glucagon-independent manner. The inventor also finds that soluble GP73 can rise fasting blood glucose in mice, and induce abnormal glucose tolerance and pyruvate tolerance. Based on the blood glucose regulation effect of GP73 described above, the inventor also proves through animal experiments: the GP73 inhibitor can reduce the blood glucose level and glycated hemoglobin level of diabetic mice and have a protective effect on islet β cells, and thereby having the effect of treating diabetes, which is achieved by the following method: treating diabetes by blocking and/or neutralizing GP73 by an anti-GP73 monoclonal antibody, down-regulating the GP73 level, or specifically reducing the GP73 expression by RNA interference.
(2) In the embodiments of the present invention, the inventor for the first time finds the binding of GP73 to glucagon and its effect on the efficacy of glucagon. Therefore, the GP73 inhibitor can also be used to prepare a drug for inhibiting glucagon. In the embodiments of the present invention, the inventor proves the existence of the GP73-glucagon complex, which has guiding significance for further studying the interaction between GP73 and glucagon.
(3) In the embodiments of the present invention, the inventor for the first time discovers that soluble GP73 can activate the glucose production in liver and a gluconeogenesis signaling pathway in a glucagon-independent manner.
(4) In the embodiments of the present invention, the inventor finds that the expression level of GP73 in diabetics is significantly higher than that in healthy people, so GP73 can be used as a marker for detecting diabetes.

### Brief Description of the Drawings

One or more embodiments are exemplarily illustrated by the corresponding figures in the drawings, and these exemplary illustrations do not constitute a limitation on the embodiments. The term "exemplary" herein means "serving as an example, embodiment or illustration". Any embodiment described herein for an "exemplary" purpose does not need to be explained as being superior to or better than other embodiments.

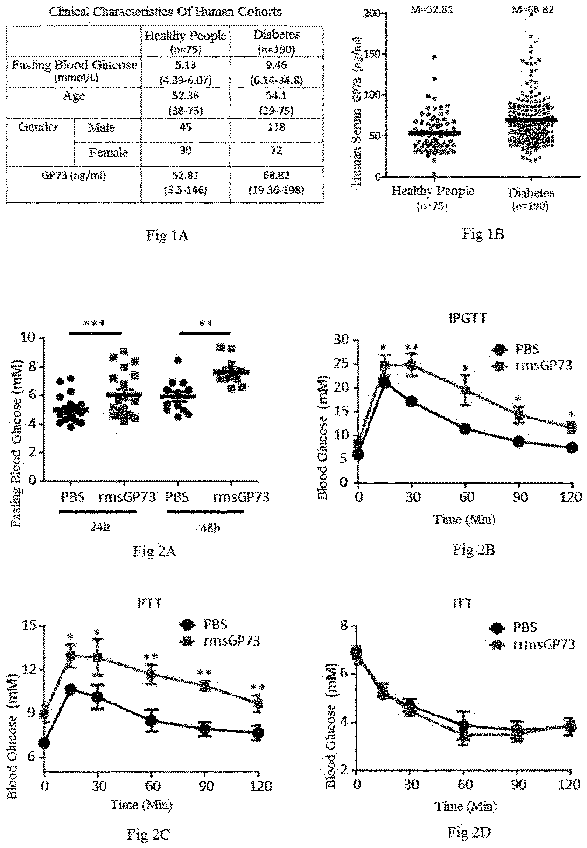

FIG. 1A shows the matching situation of genders and ages of healthy people and diabetic people before enrollment in Example 1 of the present invention. The results show that people in the two groups have no significant difference in the distribution of genders and ages. FIG. 1B shows the soluble GP73 protein levels in serum of healthy people and diabetic people in Example 1 of the present invention. The results show that the level of soluble GP73 protein in serum of diabetic people is significantly higher than that in healthy people (P<0.01).
FIG. 2A shows the fasting blood-glucose levels of mice in the recombinant mouse soluble GP73 (rmsGP73) injection experimental group and the PBS control group in Example 2 of the present invention. The results show that the fasting blood-glucose level of mice in the rmsGP73 injection experimental group is significantly higher than that in the PBS control group (^{∗}, P < 0.05; ^{∗ ∗}, P < 0.01; ^{∗ ∗ ∗}, P < 0.001). FIG. 2B shows the intraperitoneal glucose tolerance (IPGTT) levels of mice in the rmsGP73 injection experimental group and the PBS control group in Example 2 of the present invention. The results show that mice in the rmsGP73 injection experimental group show abnormalities in the IPGTT (^{∗}, P < 0.05; ^{∗∗}, P < 0.01; ^{∗∗∗}, P < 0.001). FIG. 2C shows the pyruvate tolerance (PTT) levels of mice in the rmsGP73 injection experimental group and the PBS control group in Example 2 of the present invention. The results show that mice in the rmsGP73 injection experimental group show abnormalities in PPT (^{∗}, P < 0.05; ^{∗∗}, P < 0.01; ^{∗∗∗}, P < 0.001). FIG. 2D shows the insulin tolerance (ITT) levels of mice in the rmsGP73 injection experimental group and the PBS control group in Example 2 of the present invention. The results show that mice in the rmsGP73 injection experimental group and the control group have no significant difference (^{∗}, P < 0.05; ^{∗∗}, P < 0.01; ^{∗∗∗}, P < 0.001).

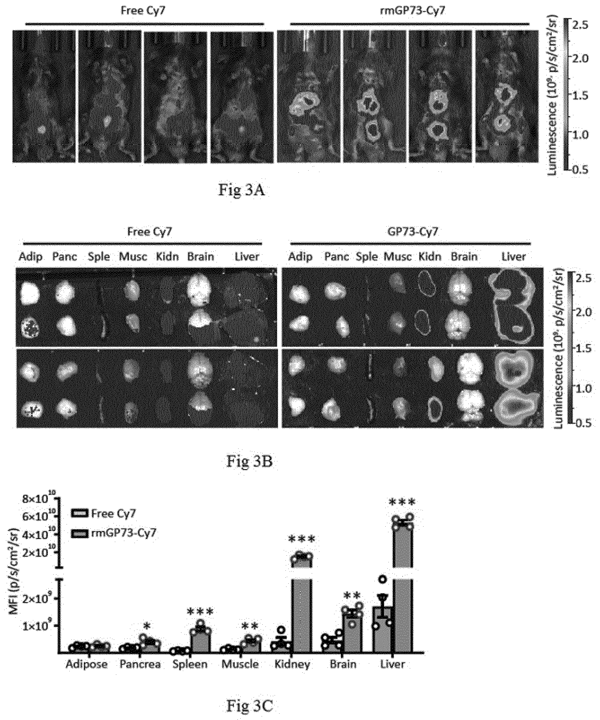

FIG. 3A shows the fluorescence distribution of intravenously injected mice in the fluorescent dye Cy7 control group and Cy7-labeled rmsGP73 protein (rmsGP73-Cy7) experimental group in Example 3 of the present invention. The results show that rmsGP73 protein aggregates obviously in liver and kidney 30 min after injection. FIG. 3B shows the distribution of fluorescence in various organs of intravenously injected mice in the Cy7 control group and the rmsGP73-Cy7 experimental group in Example 3 of the present invention. The results show that, unlike the wide distribution of unlabeled Cy7 dye, rmsGP73 protein is mainly presented in liver and kidney. FIG. 3C shows the fluorescence intensities of various organs of the intravenously injected mice in the Cy7 control group and the rmsGP73-Cy7 experimental group in Example 3 of the present invention. The results show that the fluorescence intensities of the liver, kidney and spleen in the rmsGP73-Cy7 group are significantly higher than those in the control group.

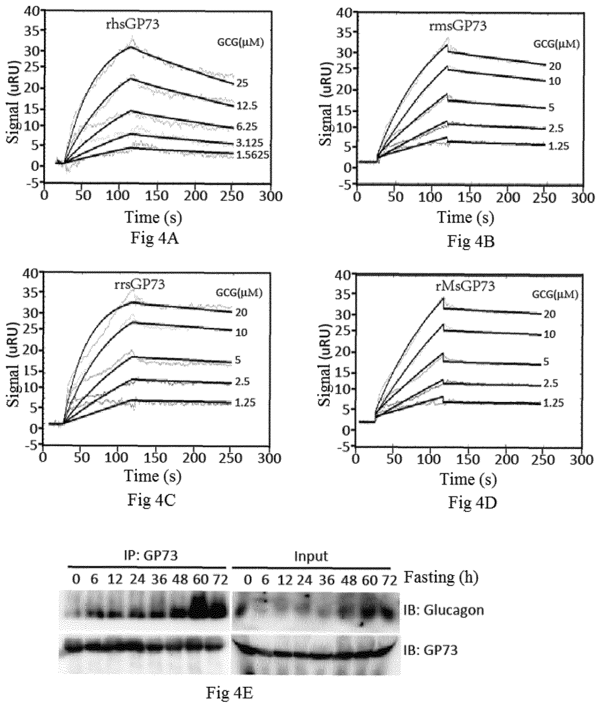

FIG. 4A shows the binding and dissociation curves of recombinant human soluble GP73 (rhsGP73) and glucagon (GCG) determined by Reichert 4SPR in Example 4 of the present invention. FIG. 4B shows the binding and dissociation curves of recombinant mouse soluble GP73 (rmsGP73) and GCG determined by Reichert 4SPR in Example 4 of the present invention. FIG. 4C shows the binding and dissociation curves of recombinant rat soluble GP73 (rrsGP73) and GCG in Example 4 of the present invention. FIG. 4D shows the binding and dissociation curves of recombinant monkey soluble GP73 (rMsGP73) and GCG determined by Reichert 4SPR in Example 4 of the present invention. FIG. 4E shows the in vivo binding ability of soluble GP73 and GCG in the co-immunoprecipitation assay in Example 4 of the present invention. The results show that there is a specific interaction between the soluble GP73 and the GCG in mouse serum, and this interaction is gradually enhanced with the prolongation of fasting time.

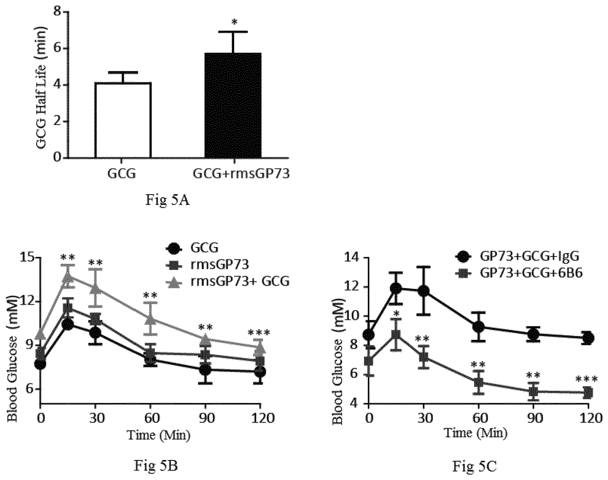

FIG. 5A shows the influence of rmsGP73 on the half-life of GCG measured in Example 5 of the present invention. The results show that rmsGP73 can significantly prolong the half-life of GCG (^{∗}, P < 0.05; ^{∗∗}, P < 0.01; ^{∗∗∗}, P < 0.001). FIG. 5B shows the blood glucose of mice in the GCG experimental group, the rmsGP73 experimental group and the rmsGP73+GCG experimental group in Example 5 of the present invention. The results show that recombinant mouse soluble GP73 protein can significantly promote the glucose-rising ability of GCG in mice (^{∗}, P < 0.05; ^{∗∗}, P < 0.01; ^{∗∗∗}, P < 0.001). FIG. 5C shows the blood glucose of mice in the rmsGP73 + GCG + IgG experimental group and the rmsGP73 + GCG + 6B6 experimental group in Example 5 of the present invention. The results show that the glucose-rising ability of GCG in mice that is promoted by rmsGP73 can be blocked by specific anti-GP73 antibody 6B6 (^{∗}, P < 0.05; ^{∗∗}, P < 0.01; ^{∗∗∗}, P < 0.001).

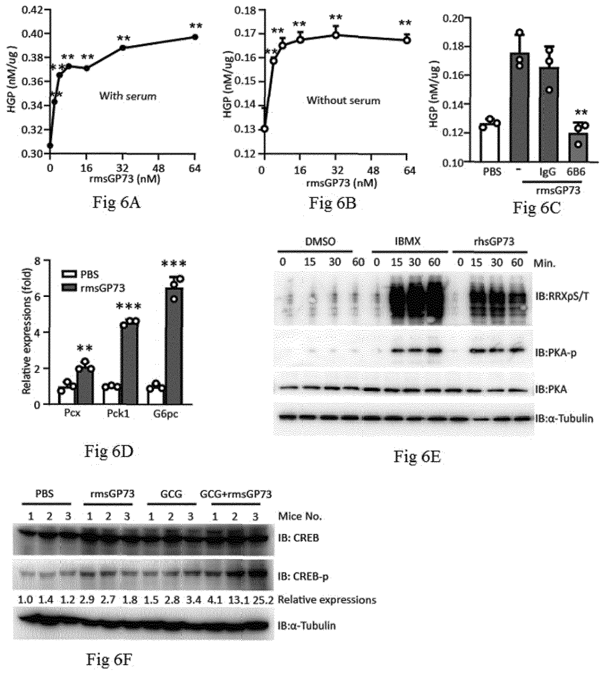

FIG. 6A shows the influence of different concentrations of rmsGP73 on the glucose production of mouse primary hepatocytes under the condition of serum culture in Example 6 of the present invention. The results show that rmsGP73 promotes the glucose production of mouse primary hepatocytes in a dose-dependent manner. FIG. 6B shows the influence of different concentrations of rmsGP73 on the glucose production of mouse primary hepatocytes under the condition of serum-free culture in Example 6 of the present invention. The results show that rmsGP73 protein directly promotes the glucose production of primary hepatocytes in a dose-dependent manner without the aid of any other hormones. FIG. 6C shows the influence of anti-GP73 antibody 6B6 on the glucose production of mouse primary hepatocytes that is promoted by rmsGP73 protein under the condition of serum-free culture in Example 6 of the present invention. The results show that 6B6 can specifically block the glucose production of primary hepatocytes promoted by rmsGP73. FIG. 6D shows the influence of rmsGP73 on the rate-limiting enzymes of gluconeogenesis in mouse primary hepatocytes under the conditon of serum-free culture in Example 6 of the present invention. The results show that rmsGP73 promotes the up-regulated expression of three key gluconeogenesis enzymes (Pckl, Pcx and G6pc). FIG. 6E shows the influence of rhsGP73 on the activity of PKA enzyme, a key kinase of gluconeogenesis signaling pathway, in HepG2 cell lines in Example 6 of the present invention. The results show that similar to the positive control IBMX (Sigma Aldrich, Art. No.: 15879), rhsGP73 promotes the phosphorylation level (PKA-p) and kinase activity (RRX ρ s/T) of PKA. FIG. 6F is an western blotting diagram of CREB-p, CREB and α-Tubulin of mouse liver tissue in the PBS control group, the GCG experimental group, the rmsGP73 experimental group and the rmsGP73 + GCG experimental group in Example 6 of the present invention. The results show that rmsGP73 can directly promote the activation of the gluconeogenesis signaling pathway and synergistically enhance the hepatic gluconeogenesis ability of GCG.

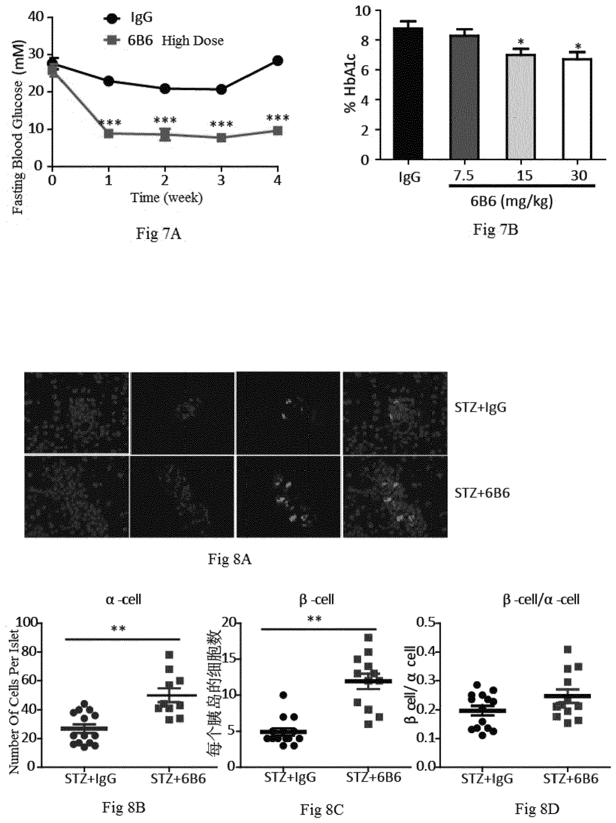

FIG. 7A shows the fasting blood-glucose levels of mice treated with IgG and anti-GP73 antibody 6B6 at 30 mg/kg respectively in Example 7 of the present invention. The results show that high-dose 6B6 significantly reduce the blood glucose of mice with type I diabetes (^{∗}, P < 0.05; ^{∗∗}, P < 0.01; ^{∗∗∗}, P < 0.001). FIG. 7B shows the glycated haemoglobin (HbAlc) levels of mice injected with IgG and 6B6 antibodies at 7.5, 15 and 30 mg/kg respectively in the fourth week after injection in Example 7 of the present invention. The results show that the middle-dose and high-dose anti-GP73 antibodies 6B6 can significantly reduce the glycated hemoglobin of mice with type I diabetes (^{∗}, P < 0.05; ^{∗∗}, P < 0.01; ^{∗∗∗}, P < 0.001).
FIG. 8A is a three-color immunofluorescent staining image of mice in the STZ+IgG group and the STZ+6B6 group in Example 8 of the present invention. The results show that the anti-GP73 antibody 6B6 has an obvious protective effect on islet α cells and islet β cells of mice with type I diabetes; FIGs. 8B-8C are statistical charts of islet α cells and islet β cells of mice in the STZ+IgG group and the STZ+6B6 group in Example 8 of the present invention. The results show that the anti-GP73 antibody 6B6 has a significant protective effect on islet α cells and islet β cells of mice with type I diabetes (^{∗}, P < 0.05; ^{∗∗}, P < 0.01; ^{∗∗∗}, P < 0.001). FIG. 8D is a diagram of ratio of islet β/α cells of mice in the STZ + IgG group and the STZ + 6B6 group in Example 8 of the present invention. The results show that the anti-GP73 antibody 6B6 has no significant effect on the ratio of β/α cells of mice with type I diabetes.

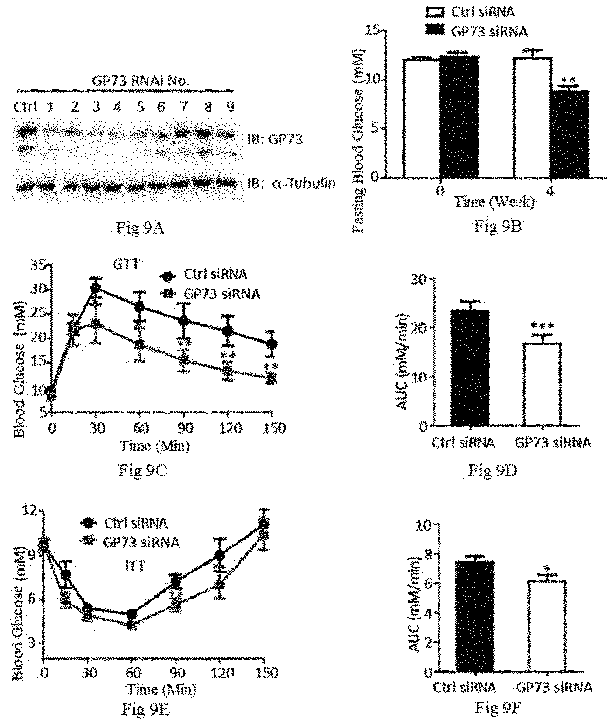

FIG. 9A shows the western blotting diagram of H22 cells transfected with 9 GP73 siRNAs in Example 9 of the present invention. The results show that different sequences have different knockdown efficiency on endogenous GP73 protein in cells, and sequence 4 is selected as the candidate SiRNA. FIG. 9B shows the fasting blood-glucose of mice in the control group (Ctr siRNA) and the GP73 siRNA group in Example 9 of the present invention in the fourth week after injection. The results show that the GP73 siRNA significantly reduces the fasting blood-glucose of mice with type II diabetes (^{∗}, P < 0.05; ^{∗∗}, P < 0.01; ^{∗∗∗}, P < 0.001). FIG. 9C shows the IPGTT levels of mice in the Ctr siRNA group and the GP73 siRNA group in the fourth week after injection in Example 9 of the present invention. The results show that GP73 siRNA obviously improves the IPGTT of mice with type II diabetes (^{∗}, P < 0.05; ^{∗∗}, P < 0.01; ^{∗∗∗}, P < 0.001). FIG. 9D shows that the influence of GP73 siRNA on the AUC value (area under the curve) of IPGTT of mice with type II diabetes in Example 9 of the present invention is significantly different (^{∗}, P < 0.05; ^{∗∗}, P < 0.01; ^{∗∗∗}, P < 0.001). FIG. 9E shows the ITT levels of mice in the Ctr siRNA group and the GP73 siRNA group in the fourth week after injection in Example 9 of the present invention. The results show that the GP73 siRNA obviously improves the ITT of mice with type II diabetes (^{∗}, P < 0.05; ^{∗∗}, P < 0.01; ^{∗∗∗}, P < 0.001). FIG. 9F shows that the influence of GP73 siRNA on the AUC values of ITT of mice with type II diabetes in Example 9 of the present invention is significantly different (^{∗}, P < 0.05; ^{∗∗}, P < 0.01; ^{∗∗∗}, P < 0.001).

### Detailed Description of the Invention

In order to make the object, technical scheme and advantages of the embodiments of the present invention clearer, the technical schemes in the embodiments of the present invention will be clearly and completely described below. Obviously, the described embodiments are part but not all of the embodiments of the present invention. Based on the embodiments of the present invention, all other embodiments obtained by those killed in the art without creative labor shall fall within the scope of protection of the present invention.

In addition, in order to better explain the present invention, numerous specific details are given in the specific embodiments below. Those skilled in the art should understand that the present invention can also be implemented without some specific details. In some embodiments, raw materials, components, methods, means, etc. well known to those skilled in the art are not described in detail in order to highlight the gist of the present invention.

Unless explicitly stated otherwise, throughout the whole specification and claims, the term "comprise" or its variations such as "comprises" or "comprising" will be understood as including the stated elements or components but not excluding other elements or components.

### I. Explanation of Terms

In the present invention, the term "GP73" refers to Golgi transmembrane glycoprotein 73 (GP73), also known as Golgi membrane protein 1 (GOLM1) or Golgi phosphorprotein 2 (GOLPH2). A proprotein convertase (PC) cleavage site is presented near the amino acid 55 of GP73. After being cleaved by PC, full-length GP73 can be released from the Golgi complex and secreted into the blood circulatory system. The GP73 fragment released into the blood is called soluble GP73. In the present invention, GP73 generally refers to natural or recombinant full-length GP73, a GP73 fragment, a GP73 mutant or modified GP73 existing in the body or isolated in vitro. Optionally, the GP73 is selected from full-length GP73 or GP73 excluding amino acids 1-55. When the term "GP73" is matched with other words, it also has the meanings as defined here. For example, GP73 in a GP73 inhibitor, an anti-GP73 antibody and an anti-GP73 monoclonal antibody has the meanings as defined here.

In the present invention, the term "GP73 inhibitor" refers to any polypeptide, protein, nucleic acid sequence or small molecule compound that can down-regulate the level (including gene level or protein level), activity, function and/or stability of GP73. Optionally, the polypeptides, protein, nucleic acid sequences or small molecule compounds that down-regulates the level, activity, function and/or stability of GP73 have one or more of the following properties: (1) inhibiting transcription, correct cutting and/or translation of genes encoding the GP73, (2) inhibiting or hindering the binding of the GP73 to receptors and/or ligands in the body, (3) inhibiting or hindering the interaction between the GP73 and specific interacting molecules in the body, and (4) shortening the half-life of the GP73 in the body. The GP73 inhibitor comprises, but is not limited to, an anti-GP73 antibody (comprising an anti-GP73 monoclonal antibody, a bispecific antibody, a multispecific antibody and antibody fusion protein), siRNA that specifically inhibits GP73, shRNA that specifically inhibits GP73, microRNA that specifically inhibits GP73, antisense oligonucleotide that specifically inhibits GP73, a nucleic acid aptamer that specifically inhibits GP73 and a small molecule compound that specifically inhibits GP73.

In the present invention, the term "antibody" refers to an immunoglobulin molecule composed of four polypeptide chains, and the four polypeptide chains refer to two heavy (H) chains and two light (L) chains linked to each other by disulfide bonds.

In the present invention, the term "monoclonal antibody" refers to a highly uniform antibody only targeting a specific epitope, which can be prepared by known methods such as hybridoma technology, antibody library technology, transgenic mouse technology or single cell PCR technology.

In the present invention, the term "chimeric antibody" refers to the use of DNA recombination technology to transform mammalian cells to express a chimeric antibody by inserting light and heavy chain variable region genes of a heterologous monoclonal antibody into an expression vector containing human antibody constant regions. The light and heavy chain variable regions in the antibody molecule expressed in this way are heterologous, while the constant regions are humanized, so nearly two thirds of the whole antibody molecule is humanized. The antibody produced in this way reduces the immunogenicity of the heterologous antibody while retaining the ability of the parent antibody to specifically bind to the antigen.

In the present invention, the term "humanized antibody" means that since FR in the variable region of the chimeric antibody still has certain immunogenicity, in order to reduce heterologous components, human FR is used to replace the heterologous FR on the basis of the chimeric antibody by using the genetic engineering technology to form a more humanized antibody. That is, except that CDR is heterologous, the rest are all humanized structures so that humanized antibody can obtain the antigen binding specificity of the mouse monoclonal antibody while reducing its heterology. Alternately, most amino acid sequences of the heterologous antibody are replaced with humanized sequences by surface remodeling and other techniques while basically retaining the affinity and specificity of the parent heterologous monoclonal antibody and reducing its heterology, thereby facilitating its application to the human monoclonal antibody.

In the present invention, the term "fully-human antibody" refers to transfer of all human genes that encode antibodies to genetically engineered antibody-gene-deficient animals by transgenic or chromosome transfer technology so that the animals can express human antibodies to obtain the fully-human antibody; or refers to a monoclonal antibody obtained by screening the human antibody library or a human monoclonal antibody obtained by the single cell PCR technology.

As used herein, the term "antigen-binding fragment of antibody" refers to a part, usually a target binding region or a variable region, of a full-length antibody.

As used herein, the terms that are not specifically explained, such as nano-antibody, heavy chain antibody, Fab, Fab-SH, Fv, scFv, F(ab')₂, DsFv, Miabody, Minibody, Tribody, Sc(Fv)₂, [Sc(Fv)₂]₂, (ScFv-SA)₄, etc. also have conventional meanings in the art.

As used herein, the term "treating" means that beneficial or desired results can be produced, comprising but not limited to: prevention, alleviation, improvement or cure of one or more symptoms, reduction of degree of illness and longer survival period than the expected survival period.

As used herein, the term "effective dose" refers to the amount that is sufficient to effectively deliver active ingredients for treating diseases when the active ingredients are administered by the method of the embodiments of the present invention, and may also refers to the amount or dose of the active ingredients that can provide expected effects to diagnosed or treated patients after being administered to the patients for single time or multiple times. The effective dose can be determined by clinicians involved as those skilled in the art through known techniques and observation results obtained in similar situations. When determining the effective amount or dose of the effective ingredients administered, the clinicians involved should consider a variety of factors, comprising but not limited to: Species, size, age and general health of mammals; the specific diseases involved; the degree of involvement or severity of the disease; individual patient's response; the specific compound administered; the administration mode; bioavailability property of the preparation administered; the selected dosage regimen; the use of therapy in combination with drug; and other related situations.

As described herein, the "pharmaceutically acceptable excipients" can be pharmaceutical carriers, excipients and other additives used in conventional preparations, such as excipients of common antibody drugs.

### II. Detection Method or Experimental Method

1. In the present invention, the detection method of soluble GP73 in human serum is as follows: 190 diabetics were randomly selected from the endocrinology departments of multiple physical examination centers in the same period, and 75 healthy people were randomly selected from multiple physical examination centers in the same period as control. Venous blood was collected from both the diabetics and the healthy people after overnight fasting, and the serum was stored in a -20 °C cryogenic refrigerator. After all the samples were collected, the soluble GP73 level in human serum was detected by a kit based on magnetic particle chemiluminescence immunoassay (purchased from Thermovision Bio).
2. In the present invention, the detection method of glucose in mice is as follows: all blood samples were collected from the tail, and the blood glucose was tested by a full-automatic glucometer (ACCU-CHEK; Roche) through a glucose oxidase method; normal mice and diabetic mice were fasting for 6 h, and fasting blood glucose was tested; the random blood glucose level was tested at 9: 00 am; and when the blood glucose level is greater than 35 mM (the upper limit of glucometer), it was recorded as 35 mM.
3. In the present invention, the detection method of the glycometabolism experiment is as follows: In the glucose, insulin and pyruvate tolerance tests, mice were fasting for 12 h and then injected with D-glucose (Sigma, Art. No.: G8270, 1.5 g/kg body weight), insulin (Sigma, Art. No.: 19278, 0.75 U/kg body weight) or sodium pyruvate (Sigma, Art. No.: P2256, 2 g/kg body weight) at the tail vein; at 0 min, 15 min, 30 min, 45 min, 60 min, 120 min and 24 h after injection, blood was collected from the tail vein of mice, and blood glucose was tested to determine the intraperitoneal glucose tolerance (IPGTT), pyruvate tolerance (PTT) and insulin tolerance (ITT).
4. In the present invention, the detection method of the GP73 glucose-rising experiment is as follows: the selected male C57BL/6N mice were fasting for 6 h and injected with rmsGP73 (80 µg/kg body weight) at the tail vein; and at 0 min, 15 min, 30 min, 60 min, 90 min and 120 min after injection, blood was collected from the tail vein of mice, and blood glucose was tested.
5. In the present invention, the detection method of the mouse in vivo imaging experiment is as follows: the selected male C57BL/6N mice were fasting overnight and the whole bodies of the mice were shaved one day in advance; rmsGP73 was labeled with Cy7 dye, and the fluorescence intensities of the Cy7 dye control and rmsGP73-Cy7 protein were adjusted to be the same using a 96-well plate; mice were injected with Cy7 dye having the adjusted fluorescence intensity and rmsGP73-Cy7 (i.e. the Cy7 control group and the rmsGP73-Cy7 experimental group, 4 mice per group, at an injection dose of 200 uL/ mouse) at the tail vein; the mice were anesthetized in an anesthesia box and subjected to mouse in vivo imaging in a in vivo imaging instrument at 0 min and 30 min after injection, and the fluorescence distribution in the mice was observed; and then, the mice were sacrificed quickly, and white fat, muscle, liver, spleen, pancreas, kidney and brain were taken out and neatly placed in a mouse imager to observe the fluorescence intensity of each organ.
6. In the present invention, the detection method of the microscale thermophoresis experiment is as follows: the recombinant soluble GP73 with a His tag was incubated with a RED-tris-NTA marker at room temperature in the dark for 30 min; GCG with 15 concentration gradients was prepared in PCR tubes, the different concentrations of GCG and the labeled GP73 protein were mixed well before incubating at room temperature for 30 min, and the above mixture liquids were sucked by a capillary tube respectively and sequentially injected into a clamping groove of a capillary column. The binding of the GP73 protein to GCG was detected using a microscale thermophoresis (NanoTemper Company) in a NT115 mode, followed by calculating the affinity value according to the fitting curve.
7. In the present invention, the detection method of the OpenSPR experiment is as follows: By coupling the recombinant soluble GP73 by an amino sensor chip (Art. No.: SEN-AU-100-3-AMINE, lot: #SAB0122, Nicoya Company) and enabling glucagon (HY-P0082, lot: #34006) with different concentration gradients to serve as the mobile phase, the binding and dissociation curves were assayed by openSPR (OpenSPR-XT, Nicoya product), and the affinity values were obtained by curve fitting.
8. In the present invention, the detection method of the immunoprecipitation experiment is as follows: mouse blood was collected from the retro-orbital venous plexus and centrifuged to obtain mouse serum; any one of an anti-mouse GP73 antibody (Santa Cruz, Art. No.: sc-365817), an anti-glucagon antibody (Abcam, Art. No.: ab92517), a RRX ρ S/T antibody (CST company, Art. No.: 9624), a PKA-p antibody (CST company, Art. No.: 5661), a α-Tubulin antibody (Sigma Aldrich, Art. No.: T9026) or a PKA antibody (CST Company, Art. No.: 5842) was added, after incubating in a shaking table at 4 °C for 1 h, agarose beads (Protein A/G PLUS-Agarose, Santa Cruz, Art. No.: sc-2003) was added, and the resulting product continued to be incubated for 2 h; the protein binding to the agarose beads was added to an SDS lysis solution and placed in boiling water bath for 10 min, and after centrifugation, the supernatant was taken for SDS-PAGE; after electrophoresis was finished, the protein on the gel was transferred to a PVDF support film by a semi-dry transfer printing system, and the resulting product was incubated in a 5% skim milk blocking buffer in a shaking table at room temperature and sealed for 1 h; according to the species specificity of the secondary antibody, a HRP-labeled goat-anti-mouse secondary antibody (ZSGB-BIO, Art. No.: ZB-2305) or HRP-labeled goat-anti-rabbit secondary antibody (Zhongshan jinqiao, Art. No.: ZB-2301) were added respectively, and incubated in a shaking table at room temperature for 1 h; and an appropriate amount of ECL chromogen solution was evenly dropped onto a PVDF membrane and imaged using a Tanon 5200 full-automatic chemiluminescent imaging analysis system.
9. In the present invention, the method for detecting the half-life of glucagon is as follows: Female C57BL/6N mice were injected with GCG (1 µg/kg body weight) or a mixture (10 min incubation at room temperature) of GCG (1 µg/kg body weight) and recombinant mouse soluble GP73 protein (1 mg/kg body weight) at the tail vein; at 0 min, 1 min, 3 min, 5 min, 10 min, 20 min and 30 min after injection, mouse blood was collected from the retro-orbital venous plexus respectively; three kinds of protease inhibitors (DPP4, Protease inhibitor cocktail and Aprotinin) were added into a collection tube according to the required concentration and mixed well quickly, and still stood at room temperature for 1 h, prior to centrifuging at 3000 rpm for 10 min to obtain mouse serum; the concentration of GCG was detected using MILLIPLEX^{®} MAP RAT METABOLIC MAGNETIC BEAD PANEL KIT 96 Well Plate Assay kit (Millipore, Art. No.: RMHMAG-84K); and the obtained data were used to calculate the pharmacokinetic parameters of a non-compartmental model, and then to calculate the half-life value in turn. GCG (purchased from MCE, Art. No.: HY-P0082, Batch No.: 34006); recombinant mouse soluble GP73 protein rmsGP73, namely the expression product of HEK293 cells in this laboratory (i.e. the recombinant mouse soluble GP73 in Example 2); protease inhibitor (Protein Inhibitor Cocktail I, purchased from Millipore, Art. No.: 20-201); DPP4 inhibitor (purchased from Millipore, Art. No.: DPP4-010), Aprotinin (purchased from Sigma, Art. No.: A6106).
10. In the present invention, the immunofluorescent staining method is as follows: after mice were sacrificed, the dissected pancreatic tissue were fixed with 10% (v/v) formalin to prepare paraffin section specimens, and paraffin sections with a thickness of 5 µm were made; the immunofluorescent sections were dewaxed to water, sealed for 30 min, and incubated with an anti-mouse insulin antibody (Abcam, Art. No.: ab181547) or anti-glucagon antibody (Abcam, Art. No.: ab10988) for 1 h, followed by washing three times; the corresponding fluorescent secondary antibody was added and incubated at room temperature for 1 h, followed by washing and DAPI staining; images were taken under a confocal fluorescence microscope (Zeiss LSM710) or an automatic digital slide scanner (3D HISTECH); and 5-10 isometric sections of each pancreas were obtained for imaging, there is at least 3 mice per group and at least three visual fields for stained positive cells, and the total number of cells is not less than 200.

### Example 1. The level of soluble GP73 in serum of diabetics is significantly higher than that of healthy people

Experimental method: In order to compare whether there is a difference in the level of soluble GP73 in serum between the diabetics and healthy people, 75 healthy people passing the physical examination in the physical examination centers and 190 diabetics diagnosed with diabetes by the endocrinology department were tested for soluble GP73 level in serum after overnight fasting. These enrolled people were subjected to age and sex matching, and the diabetics and the healthy people have no significant difference in the distribution of genders and ages. (as shown in FIG. 1A).

Experimental results: The average concentration of soluble GP73 in serum of the healthy people was 52.81 ng/mL (3.5-146 ng/mL), and the average concentration of soluble GP73 in serum of the diabetes was 68.82 ng/mL (19.36-198 ng/mL). There was a significant statistical difference between the two groups (P < 0.01) (as shown in FIG. 1B). The above results show that the level of soluble GP73 in serum of the diabetics is significantly higher than that of the healthy people.

### Example 2. Recombinant soluble GP73 regulates glycometabolism

Experimental method: GP73 protein was first expressed and purified by HEK293 cells in the mammalian cell expression system to obtain the gene recombinant mouse soluble GP73 protein (rmsGP73) (NCBI Reference Sequence: NP_001030294.1). This protein, the main existing form of soluble GP73 in blood, lacks amino acids 1-55 of GP73 (all recombinant soluble GP73 proteins used in mouse experiments in the embodiments of the present invention are recombinant mouse soluble GP73 proteins, abbreviated as rmsGP73). C57BL/6N mice are injected with rmsGP73 and PBS at 300 ng/mouse at the tail vein, respectively (i.e., the experimental group and the PBS control group, 10 mice per group). The fasting blood glucose of mice was tested 24 h and 48 h after injection (a glucometer and blood glucose test paper purchased from Roche Company). The intraperitoneal glucose tolerance (IPGTT), pyruvate tolerance (PTT) and insulin tolerance (ITT) were detected according to Part III of the detection method or experimental method.

Experimental results: The fasting blood glucose of mice in the injection experimental group was significantly higher than that in the control group (as shown in FIG. 2A). Importantly, in the metabolism experiment, compared with the control group, mice in the rmsGP73 injection experimental group showed abnormal glucose tolerance (IPGTT) (as shown in FIG. 2B) and abnormal pyruvate tolerance (PTT) (as shown in FIG. 2C), while the insulin tolerance (ITT) representing insulin sensitivity in the experimental group has no significant difference from that in the control group (as shown in FIG. 2D).

### Example 3. Recombinant soluble GP73 aggregates in mouse liver and kidney

Experimental method: The fluorescence distribution and intensity of mice after intravenous injection in the fluorescent dye Cy7 control group and the Cy7-labeled rmsGP73 protein (rmsGP73-Cy7) experimental group were tested according to Part V of the detection method or the experimental method.

Experimental results: Mouse in vivo imaging showed that compared with the Cy7 control group, rmsGP73-Cy7 mainly aggregates in the liver and kidney (as shown in FIG. 3A). After taking the mouse organs, it was found that compared with the Cy7 control group, the fluorescence intensity of rmsGP73-Cy7 in the liver and kidney was the strongest (as shown in FIG. 3B). By analyzing the fluorescence intensity of various organs of mice, it was found that the fluorescence intensity of rmsGP73-Cy7 in the liver, kidney and spleen was significantly higher than that in the Cy7 control group (as shown in FIG. 3C).

### Example 4. Specific interaction between recombinant soluble GP73 and glucagon

When the blood glucose level drops to a certain threshold, glucagon is secreted rapidly. Some amino acids, such as glutamine, arginine, alanine and free fatty acids in plasma, are strong stimulants for secretion of glucagon. Gastric inhibitory peptide (GIP) promotes the secretion of glucagon in the case of hypoglycemia. Insulin, γ-aminobutyric acid, leptin and somatostatin directly inhibit the secretion of glucagon, and glucagon-like peptide 1 (GLP-1) indirectly inhibits the secretion of glucagon. Meantime, the central nervous system is an important blood glucose level sensor, which directly or indirectly regulates the secretion of glucagon through the vagus nerves and/or cholinergic nerves. The ratio of insulin to glucagon (I/G) in blood is a key factor to control liver glycogen and gluconeogenesis. A high ratio of I/G indicates that energy is sufficient, glycogen synthesis is increased and gluconeogenesis is inhibited. Otherwise, glycogen is decomposed and gluconeogenesis is enhanced.

Hyperglucagonemia is seen in all types of diabetes. The secretion of glucagon is regulated by endogenous insulin. The loss of this regulatory mechanism leads to the body secreting more glucagon and rising blood glucose, thereby causing diabetes. In a model of type II diabetes, glucagon is a key factor in the development of hyperglycemia. Normal mice lacking a glucagon receptor exhibited symptoms of hypoglycemia, while diabetic mice lacking the glucagon receptor (*db*/*db*) showed no symptoms of hyperinsulinism or hyperglycemia. In the type II diabetes, the direct and indirect effects of insulin are impaired, while the enhanced glucagon signal further aggravates glycogen degradation and gluconeogenesis, which leads to increased glucose and rose blood glucose. Clinical studies have also shown that inappropriate secretion of glucagon is the main factor of hyperglycemia in patients with type II diabetes. Patients with type II diabetes have low insulin secretion and insulin resistance, accompanied with dysregulation of glucagon, such as increased fasting glucagon level, decreased postprandial glucagon inhibition, and reduced sensitivity of cells to blood glucose and insulin inhibiting the secretion of glucagon. Therefore, type II diabetes is generally considered as a bihormonal chaotic pancreatic disease, such as insulin deficiency, insulin resistance and excess glucagon. Mice (mice model of type I diabetes) with the glucagon receptor genes knockout (GCCR-/-) have no clinical manifestations of diabetes when destroying almost all β cells, but after the expression of GCGR in hepatocytes is restored by a adenovirus glucagon receptor (GCGR) expression vector, the blood glucose level of mice is increased rapidly. Glucagon may be a major contributor to hyperglycemia in patients with long-term type I diabetes and residual islet cells. All the research results show that glucagon is a key factor in the development of diabetes. Reducing the glucagon activity will enhance the treatment of diabetes.

### 4.1 Affinity assay of recombinant soluble GP73 of different species and GCG

Experimental method: in order to study the interaction of GP73 and glucagon (GCG, purchased from MCE company, HY-P0082), the binding activity of recombinant soluble GP73 to GCG was firstly tested by Reichert 4SPR (Life Sciences company), microscale thermophoresis (MST, nanoTemper company) and OpenSPR (OpenSPR-XT, Nicoya company).

Experimental results: Recombinant human soluble GP73 (rhsGP73) can specifically bind to GCG, and the affinity (KD) tested by the three methods of Reichert 4SPR, MST and OpenSPR was 2.83 µM, 2.45 µM and 2.80 µM respectively (as shown in FIG. 4A and Table 1). Soluble GP73 from a recombinant mouse (rmsGP73), a rat (rrsGP73) and a monkey (rMsGP73) also can specifically bind to GCG (as shown in FIGs. 4B, C and D, and Table 1). All kinds of soluble GP73 are prepared by our company through expression and purification of mammalian cells. Monkey soluble GP73: NCBI Reference Sequence: XP_011769391.1; rat soluble GP73: NCBI Reference Sequence: XP_001056825.3; mouse soluble GP73: NCBI Reference Sequence: NP_001030294.1; human soluble GP73: NCBI Reference Sequence: NP_057632.2.

**Table 1: Affinity (KD) of recombinant soluble GP73 of different species and GCG**

| Method | Affinity (µM) | | | |
|---|---|---|---|---|
| | rhsGP73 | rmsGP73 | rrsGP73 | rMsGP73 |
| Reichert 4SPR | 2.83 | 1.55 | 0.73 | 0.35 |
| MST | 2.45 | 0.41 | 0.58 | 0.12 |
| OpenSPR | 2.80 | 3.21 | 3.19 | NA |

| | | | | |
|---|---|---|---|---|
| Note: NA indicates not tested | | | | |

### 4.2 In vivo binding activity detection of soluble GP73 and GCG

Experimental method: In order to confirm the in vivo binding activity of soluble GP73 to GCG, co-immunoprecipitation assay was performed after collecting blood from C57BL/6N mice fasting for different times. Mouse anti-GP73 monoclonal antibody F12 was purchased from Santa Cruz Company.

Experimental results: GP73 precipitates in serum bind to GCG (FIG. 4E). Interestingly, the interaction band of GP73-GCG was gradually deepened, indicating that the GP73-GCG complex was gradually increased when the fasting time was longer (as shown in FIG. 4E).

### Example 5. Recombinant soluble GP73 prolongs the half-life of plasma GCG and promotes the glucose-rising effect of GCG

The aforementioned research results show that rmsGP73 can cause elevated fasting blood glucose and abnormal glucose tolerance in mice, and sGP73 can specifically bind to GCG, which suggests that sGP73 is likely to function as a molecular chaperone for GCG in vivo to prevent GCG from losing functions due to rapid degradation and/or deamidation. To this end, the influence of rmsGP73 on the half-life of glucagon was experimentally studied.

Experimental method: 12 C57BL/6N mice (8 weeks old, 20-25 g, female) were used and divided into the GCG group and the GCG + rmsGP73 group, 6 mice per group. The experimental method is detailed in Part 9 of the detection method or test method.

Experimental results: recombinant soluble GP73 can prolong the half-life of plasma glucagon (as shown in FIG. 5A and Table 2).

**Table 2: Influence of recombinant soluble GP73 on the half-life of plasma glucagon**

| Group | Half-life (min) |
|---|---|
| GCG | 4.10 ± 0.64 |
| GCG + rmsGP73 | 5.72 ± 1.83^{∗} |

| | |
|---|---|
| Compared with the GCG group, ^{∗}, P < 0.05; ^{∗ ∗}, P < 0.01; ^{∗∗∗}, P < 0.001 | |

In order to study the influence of soluble GP73 on the activity of GCG, three experimental groups of GCG, rmsGP73 and rmsGP73 + GCG were set up to perform glucose-rising experiments. The mice used in the experiments were C57BL/6N mice (6-8 weeks old, 20-25g, male), 6 mice per group. The glucometer and blood glucose test paper for blood glucose testing were purchased from Roche company.

Experimental method: GCG experimental group: mice were injected with GCG (purchased from MCE company) at 100 ng/mouse at the tail vein, and the blood glucose of the mice was tested at 0, 15, 30, 60, 90 and 120 min respectively. rmsGP73 experimental group: mice were injected with rmsGP73 protein at 100 µg/mouse at the tail vein, and the blood glucose of the mice was tested at 0, 15, 30, 60, 90 and 120 min, respectively. rmsGP73+GCG experimental group: recombinant mouse soluble GP73 at 100 µg/mouse and GCG at 100 ng/mouse were incubated in vitro for 10 min at room temperature and then injected into the tail vein of mice, and the blood glucose of the mice was tested at 0, 15, 30, 60, 90 and 120 min, respectively.

In order to further determine that soluble GP73 protein specifically targets the glucose-rising ability of GCG, an anti-GP73-specific antibody 6B6 or mouse IgG was added during the co-incubation of rmsGP73 and GCG, followed by repeating the above experiment. That is, the GCG + rmsGP73 + IgG experimental group and the rmsGP73 + GCG + 6B6 experimental group were given, and the mice are C57BL/6N mice (6-8 weeks old, 20-25g, male), 6 mice per group. At 0, 15, 30, 60, 90, and 120 min, blood was sampled to test the blood glucose of the mice, respectively.

Experimental results: After GCG was injected into the tail vein of mice, the blood glucose level rose sharply, and rmsGP73 significantly promoted the glucose-rising ability of GCG (as shown in FIG. 5B). The glucose-rising ability of GCG rmsGP73 that is promoted by mice was blocked by the specific anti-GP73 antibody 6B6 (as shown in FIG. 5C).

The anti-GP73 monoclonal antibody 6B6 used herein was obtained by immunizing animals with human soluble GP73 antigen and screening hybridoma clones with mouse soluble GP73 antigen. Specifically, the anti-GP73 monoclonal antibody 6B6 of the present invention is prepared by the following method: pure human soluble GP73 recombinant antigen (NP_057632.2) was used to immunize Balb/c mice, the spleen cells of the immunized mice were fused with mouse myeloma cells SP2/0, then mouse soluble GP73 antigen (NP_001030294.1) was used for screening a GP73-specific hybridoma monoclonal antibody, and the established stable cell lines (these hybridoma cells 6B6G6, abbreviated as 6B6, were deposited on June 20, 2019 in the China General Microbiological Culture Collection Center, located at No. 3, Courtyard No. 1, Beichen West Road, Chaoyang District, Beijing, China, with the deposite number of CGMCC NO. 18165, and the proposed classification name is: mouse hybridoma cells). The mice were intraperitoneally injected with cultured monoclonal cells, and the ascites was collected, followed by purifying the monoclonal antibody.

### Example 6. Recombinant soluble GP73 promotes activation of a hepatic gluconeogenesis signaling pathway and hepatic glucose production

Experimental method: In order to study the direct effect of soluble GP73 on hepatic gluconeogenesis and hepatic glucose production, mice were starved for 12 h, and the extracted mouse primary hepatocytes were cultured with 10% serum low-glucose DMEM medium, and placed on 6 cell culture dishes at 37 °C in a cell incubator. In the case of serum-free culture, the cells were cultured in a serum-free medium overnight after adhering. The experimental cells were cultured with serum or without serum. Before the test, the cells were washed twice with PBS, cultured in the glucose-free DMEM medium, followed by adding pyruvate and lactate gluconeogenesis raw materials. The 6 dishes of cells divided into the control group (with PBS, 3 dishes) and the experimental group (with GP73 purified protein 16 nM, 3 dishes) cultured in a 37 °C cell incubator. 20 uL medium supernatant was sucked to detect the glucose content at 0 h, 1 h, 2 h, 4 h and 8 h. Finally, the cells were collected to detect the protein content and the ratio glucose/protein, and the glucose production amount per unit cell protein was obtained. GP73 purified proteins with the concentration gradients of 0 nM, 4 nM, 8 nM, 16 nM, 32 nM and 63 nM were added in three 6-well plates and cultured in a 37 °C cell incubator. After 4h, the supernatant of the culture medium was collected to detect the glucose content, and the cells were collected to detect the protein content and the ratio glucose/protein, so that the glucose production amount per unit cell protein was obtained.

In order to study whether the rmsGP73 protein can affect the expression of key enzymes of gluconeogenesis in mouse primary hepatocytes, the above experiment was repeated. The obtained cells were washed twice with PBS and then lysed, and the cellular RNA was extracted and reverse transcribed into cDNA. According to key gluconeogenesis enzymes (Pcx, Pxkl, and G6pc), upstream and downstream primers were designed for performing qRT-PCR experiments.

To study whether recombinant human soluble GP73 protein (rhsGP73 protein) can affect the phosphorylation level and enzymatic activity of PKA (a key kinase of gluconeogenesis in hepatocytes), HepG cells were added to rhsGP73, and cell lysates were obtained at different times for western blotting experiments.

Experimental results: In the PBS control group and the rmsGP73 experimental group, in the case of serum culture, the glucose production amounts of mouse primary hepatocytes by gluconeogenesis were different. The glucose production amount of the rmsGP73 experimental group is larger than that of the control group, and was gradually increased with the increase of the dose until saturation (as shown in FIG. 6A). In the PBS control group and the rmsGP73 experimental group, in the case of serum-free culture, the glucose production amounts of mouse primary hepatocytes by gluconeogenesis were different. The glucose production amount of the rmsGP73 experimental group is larger than the control group, and was gradually increased with the increase of the dose until saturation (as shown in FIG. 6B). 6B6 can specifically inhibit GP73 promoting the glucose production of mouse primary hepatocytes by gluconeogenesis (as shown in FIG. 6C). Compared with the PBS control group, the expression levels of key gluconeogenesis enzymes, namely Pcx (phosphoenolpyruvate carboxykinase 1), Pckl (pyruvate carboxylase) and G6pc (glucose-6-phosphatase) were significantly up-regulated in the rmsGP73 group (as shown in FIG. 6D). Compared with the PBS control group, the PKA phosphorylation level (PKA-p) and the kinase activity (RRXpS/T) were significantly up-regulated in the rhsGP73 group (as shown in FIG. 6E).

In the complex regulatory network of the gluconeogenesis program, the cAMP-responsive element binding protein (CREB) is a very important regulatory factor, which promotes the gluconeogenesis through a glucagon-cAMP-PKA signaling pathway. Therefore, the phosphorylation level of CREB represents the state of gluconeogenesis.

Experimental method: In order to study the influence of rmsGP73 on gluconeogenesis in mice, C57BL/6N mice (male, 8 weeks old, 20-25 g) were divided in four groups: PBS, rmsGP73, GCG and rmsGP73 + GCG, 3 mice per group. The mice was injected with PBS, rmsGP73 (100 µg/mouse), GCG (100 ng/mouse) and rmsGP73 + GCG (rmsGP73 100 µg/mouse and GCG 100 ng/mouse, in vitro incubated for 10 min after injection) at the tail vein, and mice were sacrificed 48 h after injection to separate liver tissue for western blotting experiments. An rabbit anti-pCREB polyclonal antibody (purchased from Abcam, ab32096) and a rabbit anti-CREB monoclonal antibody (purchased from CST, #9197) were used in western blotting experiments.

Experimental results: GCG injected alone can enhance the phosphorylation level of CREB, and rmGP73 significantly enhances the GCG-activated CREB phosphorylation, which indicates that rmsGP73 interacts with GCG to enhance its hepatic gluconeogenesis ability and promote the activity of GCG (as shown in FIG. 6F). Meantime, rmsGP73 used alone can still promote hepatic gluconeogenesis, which indicates again that soluble GP73 involves the activation of the gluconeogenesis signaling pathway in GCG-dependent and GCG-independent manners.

### Example 7. The glucose-reducing effect of an anti-GP73 antibody on mice with type I diabetes

Experimental method: In order to further study the in vivo glucose-reducing effect of the 6B6 antibody, C57BL/6N mice were intraperitoneally injected with STZ to induce a mouse model of type I diabetes (6-8 weeks old, 20-25g, male). After 10 days of stabilization, the mice were randomly divided into four groups, i.e., the mouse IgG control group and the 6B6 (at 7.5, 15 and 30 mg/kg) treatment groups, 7 mice per group (the specific grouping situation is as shown in Table 3). The mice received tail vein injection daily until the end of the experiment, and the body weight and the fasting blood glucose were monitored every 7 days.

Experimental results: The body weight of the mice in the 6B6 antibody treatment group was not significantly different from that in the control group (as shown in Table 4). The 6B6 antibody significantly reduced the fasting blood glucose in mice and has a dose-dependent effect (as shown in Table 5 and FIG. 7A).

**Table 3: Grouping situation of mice with type I diabetes treated with an anti-GP73 antibody 6B6**

| Group | Treatment | Dose (mg/kg/d) | Number of injections | Route of administration | Number of animals |
|---|---|---|---|---|---|
| 1 | IgG | 15 | Once/day | Intravenous | 7 |
| 2 | 6B6 | 7.5 | Once/day | Intravenous | 7 |
| 3 | 6B6 | 15 | Once/day | Intravenous | 7 |
| 4 | 6B6 | 30 | Once/day | Intravenous | 7 |

**Table 4: Body weight of mice with type I diabetes treated with anti-GP73 antibody 6B6 at different times**

| Group | Weight (g) | | | | |
|---|---|---|---|---|---|
| | Week 0 | Week 1 | Week 2 | Week 3 | Week 4 |
| IgG | 19.92 ± 1.5 | 20.50 ± 1.5 | 20.88 ± 0.7 | 21.32 ± 33 | 20.95 ± 2.5 |
| 6B6 (low dose) | 20.93 ± 1.3 | 21.50 ± 1.6 | 21.48 ± 1.5 | 21.61 ± 1.6 | 22.58 ± 1.8 |
| 6B6 (medium dose) | 21.21 ± 1.6 | 21.62 ± 1.7 | 21.58 ± 1.7 | 21.61 ± 1.5 | 22.40 ± 2.2 |
| 6B6 (high dose) | 19.9 ± 1.7 | 20.37 ± 1.5 | 20.50 ± 1.1 | 20.61 ± 1.0 | 20.74 ± 1.3 |

**Table 5: Fasting blood glucose of mice with type I diabetes treated with an anti-GP73 antibody 6B6 at different times**

| Group | Fasting blood glucose (mmol/L) | | | | |
|---|---|---|---|---|---|
| | Week 0 | Week 1 | Week 2 | Week 3 | Week 4 |
| IgG | 27.6 ± 5.1 | 22.9 ± 3.5 | 20.9 ± 3.2 | 20.7 ± 4.1 | 28.4 ± 6.4 |
| 6B6 (low dose) | 29.6 ± 5.4 | 13.4 ± 2.9^{∗∗} | 11.3 ± 3.6^{∗∗} | 10.4 ± 3.3^{∗∗} | 12.7 + 4.3^{∗∗} |
| 6B6 (medium dose) | 24.0 ± 6.0 | 11.3 ± 3.1^{∗∗} | 11.4 ± 5.5^{∗∗} | 9.6 ± 4.6^{∗∗} | 11.0 ± 4.4^{∗∗} |
| 6B6 (high dose) | 25.8 ± 6.0 | 8.9 ± 3.5^{∗∗} | 8.6 ± 2.6^{∗∗} | 7.8 ± 3.1^{∗∗} | 9.7 ± 3.3^{∗∗} |

| | | | | | |
|---|---|---|---|---|---|
| Compared with the IgG group, ^{∗}, P < 0.05; ^{∗ ∗}, P < 0.01; ^{∗∗∗}, P < 0.001 | | | | | |

Experimental method: After 4 weeks of treatment, blood was collected to test the levels of glycated hemoglobin (HbAlc), alanine aminotransferase (ALT), aspartate aminotransferase (AST) and blood lipid. The HbAlc detection kit was purchased from Crystal Chem Company (Art. No.: 80310), and a TECAN SPARK multimode reader was used for detection. Liver function and blood lipid detection kits were purchased from RIELE Company (Art. No.: ALT191230 and AST200218), and Photometer L100 biochemical analyzer from RIELE Company was used for detection.

Experimental results: The average HbAlc level in the serum of the diabetic control mice treated with IgG was 8.78 ± 1.7%. The HbAlc levels in the diabetic mice treated with middle-dose and high-dose 6B6 antibodies were 6.99 ± 1.6 and 6.71 ± 1.5, respectively (Table 6 and FIG. 7B), which indicates that the anti-GP73 antibody 6B6 reduced the HbAlc levels in STZ-induced T1D mice in a dose-dependent manner. The results of liver function and blood lipid showed that after continuously injecting the antibody for 4 weeks, the middle-dose antibody significantly reduced the level of AST in mice (Table 6), which suggests that the antibody may have a certain protective effect on STZ-induced liver injury. In addition, the triglyceride and cholesterol levels of mice in the antibody treatment group were also significantly lower than those of mice in the control group (Table 6).

**Table 6: Changes of related biochemical indexes of mice with type I diabetes treated with an anti-GP73 antibody 6B6**

| Group | HbAlc (%) | ALT (U/L) | AST (U/L) | TG (mmol/L) | CHO (mmol/L) |
|---|---|---|---|---|---|
| IgG | 8.78 ± 1.7 | 83 ± 12 | 247 ± 29 | 2.67 ± 0.17 | 5.24 ± 0.81 |
| 6B6 (low dose) | 8.29 ± 1.3 | 76 ± 11 | 238 ± 24 | 2.61 ± 0.29 | 4.05 ± 0.58^{∗∗} |
| 6B6 (medium dose) | 6.99 ± 1.6^{∗} | 74 ± 14 | 182 ± 16^{∗∗} | 2.25 ± 0.30^{∗∗} | 3.93 ± 0.78^{∗∗} |
| 6B6 (high dose) | 6.71 ± 1.5^{∗} | 81 ± 13 | 225 ± 33 | 1.78 ± 0.23^{∗∗} | 3.51 ± 0.54^{∗∗} |

| | | | | | |
|---|---|---|---|---|---|
| Compared with the IgG group, ^{∗}, P < 0.05; ^{∗ ∗}, P < 0.01; ^{∗∗∗}, P < 0.001 | | | | | |

### Example 8. Pancreas islet protection effect of an anti-GP73 antibody on mice with type I diabetes

Experimental method: In order to further study the protective effect of GP73 blockage on STZ-induced pancreatic injury, STZ intraperitoneal injection was used for inducing a mouse model with type I diabetes. In this example, C57BL/6N mice were fasting overnight (male, 8-10 weeks old, 20-22 g) and received a single intraperitoneal injection of STZ (175 mg/kg). After 10 days of stabilization, mice were randomly divided into the mouse IgG control group and the 6B6 (24 mg/kg) experimental group, 3 mice each group, using a computer-generated randomization process based on the animals' body weight and fasting blood glucose. The mice received tail vein injection daily until the end of the experiment. Mice were sacrificed 4 weeks after administration, and the obtained pancreatic islet tissue were fixed with formalin to prepare paraffin sections which were then stained by three-color immunofluorescence staining. Among them, DAPI blue is the nucleus, insulin-stained positive β cells are green, and glucagon stained positive α cells are red.

Experimental results: After the anti-GP73 antibody was blocked, the numbers of islet α and β cells were increased by more than 1 times (FIG. 8A-C) respectively, which indicates that the anti-GP73 antibody 6B6 had an obvious protective effect on both islet α cells and β cells in mice with type I diabetes. The ratio of β cells to α cells was increased slightly from 20% to about 23%, but not statistically significant (FIG. 8D).

### Example 9. The glucose-reducing effect of RNAi that specifically blocks GP73 on mice with type II diabetes

In order to further determine the glucose-reducing effect of blocking GP73, a model of type II diabetes induced by high-fat diet was constructed, and a GP73-specific RNAi oligos knockdown way was used to study its hypoglycemic effect on mice with type II diabetes.

Experimental method: A total of 9 RNAi oligos (Table 7, SEQ ID NOs. 1-9) targeting different sites of mouse GP73 were firstly synthesized, and H22 mouse liver cells were transfected and collected for the western blotting experiments 24 h after transfection.

Experimental results: No.4 RNAi oligos sequence has a good knockdown efficiency of endogenous GP73 in cells (FIG. 9A). Subsequently, No.4 GP73 RNAi oligos (GP73 siRNA) modified by 3'-cholesterol, two-end thio-backbone and full-chain methoxy were synthesized in the Zimmer Gene Company. The in vivo stability of such chemically modified siRNA was 3-6 days, and the RNAi oligos with disrupted sequences were used as the control (CTR siRNA; Table 7).

Experimental method: On this basis, C57BL/6N mice (male, 8-10 weeks old, 20-22 g) were selected, and after feeding high-fat diet for 20 weeks, the animals were fasting for 6 h, and the fasting blood glucose was tested. When the blood glucose value was greater than 11.3 mM, it is indicated that modeling is successful. According to the fasting blood glucose, the mice were randomly divided into the Ctr siRNA group and the GP73 siRNA experimental group, 6 mice per group. The mice were first injected with 4nM at the tail vein, and then intraperitoneally injected with 4 nM, once every 5 days. The mice were sacrificed 4 weeks after the first injection, and the indicators such as blood glucose and glycated hemoglobin were detected.

Experimental results: The GP73 siRNA injection group effectively reduced the fasting blood glucose of high-fat-induced mice with type II diabetes (Table 8 and FIG. 9B). Importantly, compared with the Ctr siRNA group, the mice in the GP73 siRNA injection experimental group exhibited significantly improved glucose tolerance (FIGs. 9C-D) and increased insulin sensitivity (FIGs. 9E-F). 4 weeks after treatment, the average HbAlc level was 4.39 ± 0.67% in the serum of diabetic mice treated with Ctr siRNA in the control group and 3.37 ± 0.53% in diabetic mice treated with GP73 siRNA, respectively (Table 9), which indicates that GP73-specific siRNA can reduce the HbAlc level in high-fat-induced T2D mice. The results of liver function and blood lipid showed that after continuously injecting GP73 siRNA for 4 weeks, the AST, ALT and cholesterol levels in the serum of mice were significantly reduced, which suggests that GP73 blockage can reduce blood lipid and protect the liver function (Table 9).

**Table 7: Sequence of GP73 siRNA**

| GP73 siRNA | Sequence 5'-3' |
|---|---|
| Ctr | AUCACACCAACACAGGUCCTT |
| SEQ ID No: 1 | 275-CCUGGUGGCCUGUGUUAUUTT |
| SEQ ID No: 2 | 553-GCGAGAAGCUCAUUCGAGATT |
| SEQ ID No: 3 | 950-GCAGAAUGAGGAAACCAAUTT |
| SEQ ID No: 4 | 995-CCAACAGGCAUCCAUCCAATT |
| SEQ ID No: 5 | 1198-CAGGAGAUGAAUACGACAUTT |
| SEQ ID No: 6 | 1263 -GCAGGGAAUGACAGAAAUATT |
| SEQ ID No: 7 | 1430-UGUGAAAUGGACAGCGAAATT |
| SEQ ID No: 8 | 1802-GCUCUUACCGUCAGCAUAATT |
| SEQ ID No: 9 | 2108-GCACCUAUGGUCUGUGUUUTT |

**Table 8: Fasting blood glucose of mice with type II diabetes treated with antibodies at different times**

| Group | Fasting blood glucose (mM) | |
|---|---|---|
| | Week 0 | Week 4 |
| Ctrl siRNAi | 12.0 ± 1.7 | 12.2 ± 3.0 |
| GP73 siRNA | 12.3 ± 1.9 | 8.8 ± 0.8^{∗∗} |

| | | |
|---|---|---|
| Compared with the Ctr siRNA group, ^{∗}, P < 0.05; ^{∗ ∗}, P < 0.01; ^{∗∗∗}, P < 0.001 | | |

**Table 9: Glycosylated protein level of mice with type II diabetes treated with antibodes at different time**

| Group | HbAlc | ALT | AST | TG | CHO |
|---|---|---|---|---|---|
| Ctrl siRNA | 4.39 ± 0.67 | 77 ± 22 | 243 ± 65 | 1.27 ± 0.37 | 5.62 ± 0.97 |
| GP73 siRNA | 3.37 ± 0.53^{∗} | 56 ± 10^{∗} | 186 ± 34^{∗∗} | 1.26 ± 0.16 | 4.74 ± 1.43^{∗} |

| | | | | | |
|---|---|---|---|---|---|
| Compared with the Ctr siRNA group, ^{∗}, P < 0.05; ^{∗ ∗}, P < 0.01; ^{∗∗∗}, P < 0.001 | | | | | |

In the present invention, it is proved that the increased serum GP73 level is closely related to human diabetes and is an attractive target for the treatment of diabetes, and GP73 interacts with glucagon to promote gluconeogenesis in the liver and/or kidney in both glucagon-dependent and glucagon-independent manners. Therefore, inhibition of GP73 is an effective strategy for the treatment of diabetes.

### Industrial practicality

Through the use of a GP73 inhibitor provided in the embodiments of the present invention in the preparation of a drug for treating diabetes, the blood glucose is regulated. The inventor has also proved that through animal experiments: the GP73 inhibitor can reduce the blood glucose level and glycated hemoglobin level of diabetic mice and have a protective effect on islet β cells, and thereby having the effect of treating diabetes.

## Claims

1. Use of a GP73 inhibitor in preparation of a drug for treating diabetes and complications thereof.

2. A drug for treating diabetes and complications thereof, wherein the drug comprises a GP73 inhibitor as an active ingredient.

3. A method for treating diabetes and complications thereof, wherein the method comprises following steps: administering an effective dose of GP73 inhibitor to a subject with diabetes.

4. The use according to claim 1, the drug according to claim 2 or the method according to claim 3, wherein the diabetes comprises type I diabetes, type II diabetes and gestational diabetes.

5. The use according to claim 1, the drug according to claim 2 or the method according to claim 3, wherein treating diabetes comprises any one or more of following: (1) reducing fasting blood glucose and/or postprandial blood glucose; (2) improving glucose tolerance; (3) protecting islet α cells and/or islet β cells; (4) reducing a glucose-rising ability and/or a gluconeogenesis ability of glucagon; (5) shortening a half-life of glucagon; and (6) reducing a non-insulin-dependent glucose-rising effect and gluconeogenesis function of GP73 itself.

6. The use according to claim 1, the drug according to claim 2 or the method according to claim 3, wherein treating the complications thereof comprise any one or more of following: diabetic nephropathy, diabetic eye complications, diabetic foot, and diabetic peripheral neuropathy, wherein the diabetic eye complications comprise one or more of the following: diabetic retinopathy, uveitis related to diabetes and diabetic cataract.

7. The use according to claim 1, the drug according to claim 2 or the method according to claim 3, wherein the GP73 inhibitor comprises: polypeptides, protein, nucleic acid sequences or small molecule compounds that down-regulate a level, activity, function and/or stability of GP73; optionally, the polypeptides, protein, nucleic acid sequences or small molecule compounds that down-regulates the level, activity, function and/or stability of GP73 have one or more of following properties: (1) inhibiting transcription, correct cutting and/or translation of genes encoding the GP73, (2) inhibiting or hindering a binding of the GP73 to receptors and/or ligands in the body, (3) inhibiting or hindering an interaction between the GP73 and specific interacting molecules in the body, and (4) shortening a half-life of the GP73 in the body; and further optionally, the GP73 inhibitor comprises: one or more of an anti-GP73 monoclonal antibody or an antibody fragment comprising an antigen-binding site thereof, a fusion protein of the anti-GP73 monoclonal antibody or the antibody fragment comprising the antigen-binding site thereof, and a nucleic acid sequence that specifically inhibits the GP73.

8. The use, the drug or the method according to claim 5, wherein the GP73 is selected from one or more of the following: natural or recombinant full-length GP73, a GP73 fragment, a GP73 mutant or a modified GP73 existing in the body or isolated in vitro; optionally, the GP73 is selected from full-length GP73 or GP73 excluding amino acids 1-55.

9. The use, the drug or the method according to claim 5, wherein the anti-GP73 monoclonal antibody is selected from: one or more of a monoclonal antibody produced by hybridoma cells, a monoclonal antibody screened by an antibody library, a monoclonal antibody produced by single cell PCR, a genetically engineered monoclonal antibody, a heterologous antibody, a chimeric antibody, a humanized antibody, an fully-human antibody, Nanobody and Heavy Chain Antibody; and/or, the antibody fragment is selected from: one or more of Fab, Fab-SH, Fv, scFv, F(ab')₂, DsFv, Diabody, Minibody, Tribody, Sc(Fv)₂, [Sc(Fv)₂]₂ and (ScFv-SA)₄;
and/or, the nucleic acid that specifically inhibits GP73 comprises one or more of siRNA, shRNA, microRNA, antisense oligonucleotide, miRNA and a nucleic acid aptamer; optionally, the siRNA that specifically inhibits GP73 is selected from one or more of nucleotide sequences shown in SEQ ID NO: 1 to SEQ ID NO: 9, or is selected from a sequence having at least 60%, 70%, 80% and 90% homology to any one of the nucleotide sequence shown in SEQ ID NO: 1 to SEQ ID NO: 9. and further optionally, the siRNA that specifically inhibits GP73 is selected from the nucleotide sequence shown in SEQ ID NO: 4 or the sequence having at least 60%, 70%, 80% and 90% homology thereto.

10. The use according to claim 1, the drug according to claim 2 or the method according to claim 3, wherein the drug for treating diabetes and complications thereof also comprises other drugs for treating diabetes; optionally, the other drugs for treating diabetes are selected from one or more of insulin, dimethyl biguanide, sulfonylurea hypoglycemic drugs, α- glycosidase inhibitors, thiazolidinediones, dipeptidyl peptidase 4 (DPP4) inhibitors, glucagon-like peptide-1 (GLP-1) analogs and SGLT2 inhibitors.

11. Use of a GP73 inhibitor in preparation of a drug for inhibiting glucagon.

12. A drug for inhibiting glucagon, wherein the drug comprises a GP73 inhibitor.

13. A method for inhibiting glucagon, wherein the method comprises following steps: administering an effective dose of a GP73 inhibitor to a subject in need of glucagon inhibition.

14. The use according to claim 11, the drug according to claim 12 or the method according to claim 13, wherein the glucagon inhibition comprises any one or more of following: (1) shortening a half-life of glucagon; and (2) reducing a glucose-rising ability and/or a gluconeogenesis ability of glucagon.

15. The use according to claim 11, the drug according to claim 12 or the method according to claim 13, wherein the GP73 inhibitor comprises: polypeptides, protein, nucleic acid sequences or small molecule compounds that down-regulate a level, activity, function and/or stability of GP73; optionally, the polypeptides, protein, nucleic acid sequences or small molecule compounds that down-regulates the level, activity, function and/or stability of GP73 have one or more of following properties: (1) inhibiting transcription, correct cutting and/or translation of genes encoding the GP73, (2) inhibiting or hindering a binding of the GP73 to receptors and/or ligands in the body, (3) inhibiting or hindering an interaction between the GP73 and specific interacting molecules in the body, and (4) shortening a half-life of the GP73 in the body; and further optionally, the GP73 inhibitor comprises: one or more of an anti-GP73 monoclonal antibody or an antibody fragment comprising an antigen-binding site thereof, a fusion protein of the anti-GP73 monoclonal antibody or the antibody fragment comprising the antigen-binding site thereof, and a nucleic acid sequence that specifically inhibits the GP73.

16. The use according to claim 11, the drug according to claim 12 or the method according to claim 13, wherein the GP73 is selected from one or more of following: natural or recombinant full-length GP73, a GP73 fragment, a GP73 mutant or modified GP73 existing in the body or isolated in vitro; optionally, the GP73 is selected from full-length GP73 or GP73 excluding amino acids 1-55.

17. The use according to claim 11, the drug according to claim 12 or the method according to claim 13, wherein the anti-GP73 monoclonal antibody is selected from: one or more of a monoclonal antibody produced by hybridoma cells, a monoclonal antibody screened by an antibody library, a monoclonal antibody produced by single cell PCR, a genetically engineered monoclonal antibody, a heterologous antibody, a chimeric antibody, a humanized antibody, an fully-human antibody, Nanobody and Heavy Chain Antibody;
and/or, the antibody fragment is selected from: one or more of Fab, Fab-SH, Fv, scFv, F(ab')₂, DsFv, Diabody, Minibody, Tribody, Sc(Fv)₂, [Sc(Fv)₂]₂ and (ScFv-SA)₄;
and/or, the nucleic acid that specifically inhibits GP73 comprises one or more of siRNA, shRNA, microRNA, antisense oligonucleotide, miRNA and a nucleic acid aptamer; optionally, the siRNA that specifically inhibits GP73 is selected from one or more of nucleotide sequences shown in SEQ ID NO: 1 to SEQ ID NO: 9, or is selected from a sequence having at least 60%, 70%, 80% and 90% homology to any one of the nucleotide sequence shown in SEQ ID NO: 1 to SEQ ID NO: 9; and further optionally, the siRNA that specifically inhibits GP73 is selected from the nucleotide sequence shown in SEQ ID NO: 4 or the sequence having at least 60%, 70%, 80% and 90% homology thereto.

18. A GP73-glucagon complex, wherein GP73 binds to glucagon; the GP73 is selected from one or more of following: natural or recombinant full-length GP73, a GP73 fragment, a GP73 mutant or modified GP73 existing in the body or isolated in vitro; optionally, the GP73 is selected from full-length GP73 or GP73 excluding amino acids 1-55; further optionally, a species source of the GP73 is selected from one or more of human, mice, rats, monkeys, rabbits, pigs and dogs.

19. A method for determining a binding epitope of GP73 to glucagon, comprising following steps: the binding epitope of GP73 to glucagon is determined by one or more of complex crystallization analysis method, epitope determination site excision method, hydrogen tritium exchange method and peptide-panning method.

20. A method for determining a strength of an inhibitory effect of a GP73 inhibitor on inhibiting formation of a GP73-glucagon complex, comprising any one of following two methods:
Method 1:
after incubating a candidate GP73 inhibitor with GP73, a resulting mixture and/or complex binds to glucagon, and
comparing a binding ability of the GP73 to the glucagon before and after incubating with the candidate GP73 inhibitor;
Method 2:
comparing the binding ability of GP73 to the glucagon before and after incubating with the candidate GP73 inhibitor by using a computer simulation.

21. The method according to claim 20, wherein the candidate GP73 inhibitor is derived from one or more selected from a group consisting of a hybridoma cell, a B cell, a memory B cell, an antibody library, a compound library, a GP73 analog and a glucagon analog;
and/or, the method for determining the binding ability of GP73 to glucagon comprises one or more of following methods: surface plasmon resonance (SPR) assay, microscale thermophoresis (MST) assay, and competitive ELISA assay.

22. Use of a serum-soluble GP73 detection reagent in preparation of a reagent for detecting diabetes.

23. The use according to claim 22, wherein the diabetes comprises type I diabetes, type II diabetes and gestational diabetes.

24. The use according to claim 22, wherein the GP73 detection reagent comprises a reagent for detecting a soluble GP73 in serum.

25. Use of a GP73 inhibitor in preparation of a drug for a gluconeogenesis signaling pathway inhibitor.

26. A drug for inhibiting a gluconeogenic signaling pathway, wherein the drug comprises a GP73 inhibitor.

27. A method for inhibiting a gluconeogenesis signaling pathway, wherein the method comprises a following step: administering an effective dose of a GP73 inhibitor to a subject in need of inhibiting the gluconeogenesis signaling pathway.

28. The use according to claim 25, the drug according to claim 26 or the method according to claim 27, wherein inhibiting the gluconeogenesis signaling pathway comprises any one or more of following: (1) inhibiting gluconeogenesis of hepatocytes to produce glucose; (2) down-regulating an expression level of key gluconeogenesis enzymes Pcx, Pckl and G6pc; and (3) down-regulating a PKA phosphorylation level and kinase activity.

29. The use according to claim 25, the drug according to claim 26 or the method according to claim 27, wherein the GP73 inhibitor comprises: polypeptides, protein, nucleic acid sequences or small molecule compounds that down-regulate a level, activity, function and/or stability of GP73; optionally, the polypeptides, protein, nucleic acid sequences or small molecule compounds that down-regulates the level, activity, function and/or stability of GP73 have one or more of following properties: (1) inhibiting transcription, correct cutting and/or translation of genes encoding the GP73, (2) inhibiting or hindering a binding of the GP73 to receptors and/or ligands in the body, (3) inhibiting or hindering an interaction between the GP73 and specific interacting molecules in the body, and (4) shortening a half-life of the GP73 in the body; and further optionally, the GP73 inhibitor comprises: one or more of an anti-GP73 monoclonal antibody or an antibody fragment comprising an antigen-binding site thereof, a fusion protein of the anti-GP73 monoclonal antibody or the antibody fragment comprising the antigen-binding site thereof, and a nucleic acid sequence that specifically inhibits the GP73.

30. The use according to claim 25, the drug according to claim 26 or the method according to claim 27, wherein the GP73 is selected from one or more of following: natural or recombinant full-length GP73, a GP73 fragment, a GP73 mutant or modified GP73 existing in the body or isolated in vitro; optionally, the GP73 is selected from full-length GP73 or GP73 excluding amino acids 1-55.

31. The use according to claim 25, the drug according to claim 26 or the method according to claim 27, wherein the anti-GP73 monoclonal antibody is selected from: one or more of a monoclonal antibody produced by hybridoma cells, a monoclonal antibody screened by an antibody library, a monoclonal antibody produced by single cell PCR, a genetically engineered monoclonal antibody, a heterologous antibody, a chimeric antibody, a humanized antibody, an fully-human antibody, Nanobody and Heavy Chain Antibody;
and/or, the antibody fragment is selected from: one or more of Fab, Fab-SH, Fv, scFv, F(ab')₂, DsFv, Diabody, Minibody, Tribody, Sc(Fv)₂, [Sc(Fv)₂]₂ and (ScFv-SA)₄;
and/or, the nucleic acid that specifically inhibits GP73 comprises one or more of siRNA, shRNA, microRNA, antisense oligonucleotide, miRNA and a nucleic acid aptamer; optionally, the siRNA that specifically inhibits GP73 is selected from one or more of nucleotide sequences shown in SEQ ID NO: 1 to SEQ ID NO: 9, or is selected from a sequence having at least 60%, 70%, 80% and 90% homology to any one of the nucleotide sequence shown in SEQ ID NO: 1 to SEQ ID NO: 9; and further optionally, the siRNA that specifically inhibits GP73 is selected from the nucleotide sequence shown in SEQ ID NO: 4 or the sequence having at least 60%, 70%, 80% and 90% homology thereto.
